(19) Europäisches Patentamt
European Patent Office
Office européen des brevets

(11) **EP 2 225 387 B1**

(12) **EUROPEAN PATENT SPECIFICATION**

(45) Date of publication and mention
of the grant of the patent:
**12.09.2012 Bulletin 2012/37**

(51) Int Cl.:
*C12P 19/00* [(2006.01)]     *C12P 7/06* [(2006.01)]
*C12P 7/56* [(2006.01)]

(21) Application number: **08868615.9**

(22) Date of filing: **16.12.2008**

(86) International application number:
**PCT/JP2008/073265**

(87) International publication number:
**WO 2009/084492 (09.07.2009 Gazette 2009/28)**

(54) **Process for producing saccharide**

Verfahren zur Saccharidherstellung

Procédé de production de saccharide

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.12.2007  JP 2007338059**
**27.12.2007  JP 2007338060**
**11.08.2008  JP 2008207290**
**15.08.2008  JP 2008209154**

(43) Date of publication of application:
**08.09.2010  Bulletin 2010/36**

(73) Proprietor: **Kao Corporation**
**Chuo-ku**
**Tokyo 103-8210 (JP)**

(72) Inventors:
• **NOJIRI, Naoki**
**Wakayama-shi**
**Wakayama 640-8580 (JP)**
• **UMEHARA, Masahiro**
**Wakayama-shi**
**Wakayama 640-8580 (JP)**
• **TOMIOKA, Keiichiro**
**Wakayama-shi**
**Wakayama 640-8580 (JP)**
• **KAWANO, Takako**
**Haga-gun**
**Tochigi 321-3497 (JP)**
• **KITSUKI, Tomohito**
**Wakayama-shi**
**Wakayama 640-8580 (JP)**
• **OKUTSU, Munehisa**
**Wakayama-shi**
**Wakayama 640-8580 (JP)**

• **OGAWA, Akinori**
**Haga-gun**
**Tochigi 321-3497 (JP)**

(74) Representative: **HOFFMANN EITLE**
**Patent- und Rechtsanwälte**
**Arabellastrasse 4**
**81925 München (DE)**

(56) References cited:
**WO-A-2008/099929**

• **KOBAYASHI ET AL: "A new pulverized biomass utilization technology" POWDER TECHNOLOGY, vol. 180, 12 March 2007 (2007-03-12), pages 272-283, XP022406245**
• **ZHANG ET AL: "Morphological and structural development of hardwood cellulose during mechanochemical pretreatment in solid state through pan-milling" CELLULOSE, vol. 14, 27 June 2007 (2007-06-27), pages 447-456, XP019524881**
• **KOBAYASHI ET AL: "Evaluation of wood powder property pulverized by a vibration mill" JOURNAL OF THE JAPANESE INSTITUTE OF ENERGY, vol. 86, September 2007 (2007-09), pages 730-735, XP002529673**
• **YOSHIDA ET AL: "Effects of cellulose crystallinity, hemicellulose, and lignin on the enzymatic hydrolysis of Miscanthus sinensis to monosaccharides" BIOSCIENCE, BIOTECHNOLOGY AND BIOCHEMISTRY, vol. 72, 7 March 2008 (2008-03-07), pages 805-810, XP002529666**

- INOUE ET AL: "Combining hot-compressed water and ball milling pretreatments to improve the efficiency of the enzymatic hydrtolysis of eucalyptus" BIOTECHNOLOGY FOR BIOFUELS, vol. 1, 15 April 2008 (2008-04-15), pages 1-9, XP002529503 Retrieved from the Internet: URL: www.pubmedcentral.nih.gov/picrender.fc gi?artid=2375867&blobtype=pdf>
- MAIS ET AL: "Enhancing the enzymatic hydrolysis of cellulosic materials using simultaneous ball milling", APPLIED BIOCHEMISTRY AND BIOTECHNOLOGY, vol. 98-100, 2002, pages 815-832, XP008060476,
- PARK ET AL: "Cellulose crystallinity index: measurement techniques and their impact on interpreting cellulase performance", BIOTECHNOLOGY FOR BIOFUELS, vol. 3, 2010, pages 1689-1705, XP007915903,
- CHRISTAKOPOULOS ET AL: "Direct conversion of straw to ethanol by Fusarium oxysporum: effect of cellulose crystallinity", ENZYME AND MICROBIAL TECHNOLOGY, vol. 13, 1991, pages 272-274, XP023791210,
- TASSINARI ET AL: "Energy requirements and process design considerations in compression-milling pretreatment of cellulosic wastes for enzymatic hydrolysis", BIOTECHNOLOGY AND BIOENGINEERING, vol. XXII, 1980, pages 1689-1705, XP007919686,

**Description**

FIELD OF THE INVENTION

The present invention relates to a process for producing saccharide.

BACKGROUND OF THE INVENTION

Celluloses obtained by milling cellulose-containing raw materials such as pulp have been used as industrial materials, such as raw materials of cellulose ethers, cosmetics, foodstuffs, and biomass materials. In recent years, from the viewpoint of, for example, solving environmental problems, attempts have been made to produce saccahride from a biomass material and to convert the saccahride to ethanol or lactic acid through a fermentation technique. In the production of saccharide from a biomass material by use of enzymes such as a cellulase, to obtain cellulose whose cellulose crystal structure is decrystallized in a preliminary treatment step is especially useful. For example, JP 2006-223152A discloses that cellulose is decrystallized by use of a cellulose-solvent such as lithium chloride/dimethylacetamide.

Also, there is known a method of mechanically treating pulp by means of a mill to reduce the crystallinity of cellulose in the pulp.

In Examples 1 and 4 of JP 62-236801A, there is disclosed a method of treating sheet-like pulp using a vibration ball mill or a twin-screw extruder. In Examples 1, to 3 of JP 2003-64184A, there is disclosed a method of treating pulp using a ball mill. In Examples 1 and 2 of JP 2004-331918A, there is disclosed a method of treating cellulose powders obtained by subjecting pulp to chemical treatments such as hydrolysis using a ball mill and further an air mill. JP 2005-68140A discloses a method of treating pulp kept dispersed in water using a media-type mill such as a vibration ball mill.

However, these methods have failed to achieve satisfactory efficiency and productivity when the crystallinity of celluloses is reduced.

Meanwhile, JP 2003-135052A discloses a method of saccharifying cellulose employing a specific cellulase. JP 2007-74992A and JP 2007-74993A disclose saccharification methods in which cellulose or hemicellulose has been treated with hot water by using hydrogen peroxide, followed by an enzyme treatment.

Zhang et al. (Cellulose 14, 2007, 447-456) discuss the morphological and structural development of hardwood cellulose during mechanochemical pretreatment in solid state through pan-milling. The article states that pulverizing or milling cellulose reduces its crystallinity, thereby making it more soluble and more accessible for chemical modification. Specifically, the increased reactivity is indicated to be due to the presence of more free OH groups in the milled cellulose.

Kobayashi et al. (Powder Technology, 180, 2007, 272-283) concerns pulverized wood biomass utilization technologies, such as gasification and liquefaction. The article indicates that the crystallinity of cellulose can be reduced by milling, thereby increasing the yield of saccharine when treating the cellulose with hot compressed water.

Kobayashi et al. (Journal of the Japan Institute of Energy 86, 2007, 730-735) evaluates the properties of wood powder pulverized by a vibration mill. Among others, the relationship between particle size and crystallinity is analyzed.

Christakopoulos et al. (Enzyme Microb. Technol., 1991, vol. 13, pp. 272-4) describes the fermentation of wheat straw to ethanol by *Fusarium oxysporum F3* in a one-step process. Ethanol yields were reported to increase linearly with decreasing crystallinity index. Approximately 80% of strw carbohydrates were converted directly to ethanol with a yield of 0.28 g ethanol/g of straw when the crystallinity index was reduced to 23.6%.

Tassinari et al. (Biotechnology and Bioengineering, 1980, Vol. XXII, pp 1689-1705) report that compression-milling pretreatment of lignocellulosics is effective for enzymatic hydrolysis of a variety of substrate sources. Reductions in the degree of crystallinity and the degree of polymerization of cellulose and partial destruction of the structural integrity of lignocellulosics brought about by compression-milling is reported to significantly increase the susceptibility of cellulose to enzymatic hydrolysis.

However, these methods are unsatisfactory in terms of saccharification efficiency and productivity.

SUMMARY OF THE INVENTION

The present invention relates to a process for producing saccharide, including saccharifying decrystallized cellulose prepared from a raw material containing cellulose having cellulose 1-type crystallinity of more than 33% as calculated from the following formula (1):

$$\text{Cellulose I-type Crystallinity (\%)} = [(I_{22.6} - I_{18.5})$$

$$/I_{22.6}] \times 100 \qquad (1),$$

wherein $I_{22.6}$ is diffraction intensity of a lattice plane (002 plane) as measured at a diffraction angle 2θ of 22.6° in X-ray diffraction analysis; and $I_{18.5}$ is diffraction intensity of an amorphous moiety as measured at a diffraction angle 2θ of 18.5° in X-ray diffraction analysis, the process containing:

treating the cellulose-containing raw material by use of a mill to reduce the cellulose I-type crystallinity of the cellulose to 10% or less, wherein the cellulose-containing raw material has a cellulose content of a residue obtained by removing water from the cellulose-containing raw material of 20% by weight or more, to thereby prepare decrystallized cellulose, and causing a cellulase and/or a hemicellulase to act on the decrystallized cellulose, to thereby saccharify the decrystallized cellulose , wherein the celluose-containing raw material has a bulk density of 100 to 500 kg/m$^3$ and an average particle size of 0.01 to 1mm.

DETAILED DESCRIPTION OF THE INVENTION

The present invention relates to a process for producing saccharide, including an enzymatic reaction between a cellulase or a similar enzyme and decrystallized cellulose, as a substrate, having reduced cellulose I-type crystallinity and obtained from a cellulose-containing raw material. The process can produce saccharide in an efficient manner with excellent productivity.

The present inventors have found that the aforementioned problems can be solved by performing a preliminary treatment (decrystallization) of a specific cellulose-containing raw material as a starting material by means of a mill and, subsequently, performing an enzymatic reaction by use of, for example, a cellulase.

Thus, the present invention relates to a process for producing saccharide, including saccharifying decrystallized cellulose prepared from a raw material containing cellulose having cellulose I-type crystallinity of more than 33% as calculated from the following formula (1):

$$\texttt{Cellulose I-type Crystallinity (\%) = [(I_{22.6} - I_{18.5})}$$

$$\texttt{/I_{22.6}] } \times \texttt{ 100 \quad\quad (1),}$$

wherein $I_{22.6}$ is diffraction intensity of a lattice plane (002 plane) as measured at a diffraction angle 2θ of 22.6° in X-ray diffraction analysis; and $I_{18.5}$ is diffraction intensity of an amorphous moiety as measured at a diffraction angle 2θ of 18.5° in X-ray diffraction analysis, the process containing:

treating the cellulose-containing raw material using a mill to reduce the cellulose I-type crystallinity of the cellulose to 10% or less, wherein the cellulose-containing raw material has a cellulose content of a residue obtained by removing water from the cellulose-containing raw material of 20% by weight or more, to thereby prepare decrystallized cellulose, and
causing a cellulase and/or a hemicellulase to act on the decrystallized cellulose, to thereby saccharify the decrystallized cellulose, wherein the celluose-containing raw material has a bulk density of 100 to 500 kg/m$^3$ and an average particle size of 0.01 to 1mm.

Hereinafter, the present invention will next be described in detail. As used herein, the term "cellulose I-type crystallinity" may be referred to simply as "crystallinity."

[Cellulose-Containing Raw Material]

The cellulose content of a residue obtained by removing water from the cellulose-containing raw material used in the present invention, is 20% by weight or more, preferably 40% by weight or more and more preferably 60% by weight or more.

The cellulose content used in the present invention means a total content of cellulose and hemicellulose.

The cellulose-containing raw material used in the present invention is not particularly limited. Examples of the cellulose-containing raw material include wood materials such as various wood chips, pruned-off branches, thinning wastes, and branches; pulp such as wood pulp produced from wood materials, and cotton linter pulp obtained from fiber surrounding cotton seeds; paper such as newspaper, corrugated cardboard, magazine, and wood-free paper; stems or leaves of plants such as rice straw, and corn stems; and shells of plants such as chaff; palm shells, and coconut shells. Among them, pulp, paper, stems or leaves of plants, shells of plants, and wood materials are preferred, with pulp and paper being more preferred.

In commercially available pulp products, the cellulose content of a residue obtained by removing water therefrom generally ranges from 75 to 99% by weight, and the pulp may also contain lignin, etc., as the other components. Further, commercially available pulp usually has cellulose I-type crystallinity of 60% or more.

The water content of the cellulose-containing raw material is preferably 20% by weight or less, more preferably 15% by weight or less, even more preferably 10% by weight or less. When the water content of the cellulose-containing raw material is 20% by weight or less, the raw material is readily milled, and the crystallinity thereof is readily reduced by the below-mentioned milling treatment, whereby the subsequent production of saccharide can be performed at high efficiency.

[Cellulose I-Type Crystallinity]

The decrystallized cellulose prepared according to the present invention has reduced cellulose I-type crystallinity of 10% or less. The cellulose I-type crystallinity is calculated from diffraction intensity values measured by X-ray diffraction analysis according to the Segal method, and is defined by the following calculation formula (1):

$$\text{Cellulose I-type Crystallinity (\%)} = [(I_{22.6} - I_{18.5}) / I_{22.6}] \times 100 \qquad (1)$$

wherein $I_{22.6}$ is diffraction intensity of a lattice plane (002 plane) as measured at a diffraction angle $2\theta$ of 22.6° in X-ray diffraction analysis; and $I_{18.5}$ is diffraction intensity of an amorphous moiety as measured at a diffraction angle $2\theta$ of 18.5° in X-ray diffraction analysis.

A crystallinity of 33% or less enhances the chemical reactivity of cellulose. For example, when an alkali is added to cellulose upon production of cellulose ethers, conversion of the cellulose into alkali cellulose can readily proceed, resulting in enhanced reaction conversion rate in an etherification reaction of the cellulose. From this viewpoint, the crystallinity of the decrystallized cellulose is 10% or less, most preferably 0%, indicating that no cellulose I-type crystal is detected upon analysis of the cellulose. Meanwhile, the cellulose I-type crystallinity defined by the above calculation formula (1) might be sometimes calculated as a negative value (minus value). The cellulose I-type crystallinity expressed by such a minus value is regarded as 0%.

The cellulose I-type crystallinity used herein means a ratio of the I-type crystal of cellulose on the basis of a total amount of a crystalline region of the cellulose. Also, the cellulose I-type means a crystal structure of natural cellulose. The crystallinity of the cellulose has some relation to physical and chemical properties thereof. As crystallinity increases, hardness, density, etc. of cellulose increase, by virtue of high crystallinity and a less amorphous moiety thereof, but elongation, softness, solubility in water or solvents, and chemical reactivity are lowered.

[Decrystallization Treatment]

In the present invention, the cellulose-containing raw material having a bulk density of 100 to 500 kg/m$^3$ and an average particle size of 0.01 to 1 mm is treated by means of a mill to reduce cellulose I-type crystallinity of the cellulose contained therein to 10% or less. When a cellulose-containing raw material having a bulk density less than 100 kg/m$^3$ is employed, a preliminary treatment is preferably performed, to thereby increase bulk density to 100 to 500 kg/m$^3$. Thus, through treating the cellulose-containing raw material by means of the mill, the raw material can be further milled to reduce the crystallinity thereof, thereby efficiently performing decrystallization of the cellulose.

In the present invention, a media-type mill is preferably used as a mill. Media-type mills are classified into container-driving-type mills and media-agitating-type mills. Examples of container-driving-type mills include a ball mill, a vibration mill, a planetary mill, and a centrifugal fluid mill. Among these container driving-type mills, from the viewpoints of good grinding efficiency and a good productivity, a vibration mill is preferred. Examples of media-agitating-type mills include tower-type mills such as a tower mill; agitation-tank-type mills such as an Attritor, an Aquamizer, and a Sand grinder; flow-tank-type mills such as a Visco mill and a Pearl mill; flow-tube-type mills; annular-type mills such as a co-ball mill; and continuous-type dynamic mills. Among these media-agitating-type mills, from the viewpoints of high grinding efficiency and good productivity, agitation tank-type mills are preferred. When a media agitating-type mill is employed, the peripheral speed of agitation blades thereof is preferably 0.5 to 20 m/s, more preferably 1 to 15 m/s.

The above types of mills will be understood by referring to "Progress of Chemical Engineering; 30th Collection; Control of Microparticles", Institute of Chemical Engineering, Tokai Division, October 10, 1996, Maki-Shoten.

The treatment method may be either a batch method or a continuous method.

Examples of the media (grinding media) used in the mills include balls, rods, and tubes. Among these media, from the viewpoints of high grinding efficiency and good productivity, preferred are balls and rods.

The material of the media used in the mills is not particularly limited. Examples of the material of the media include iron, stainless steel, alumina, zirconia, silicon carbide, silicon nitride, and glass.

When a vibration mill is employed as the mill and balls as the media therefor, the outer diameter of the balls is preferably 0.1 to 100 mm, more preferably 0.5 to 50 mm. When the size of the balls falls within the above specified range, desired grinding force can be attained, and the cellulose can be efficiently decrystallized without contamination of the cellulose-containing raw material which would otherwise be caused by inclusion of fragments of the balls thereinto.

According to the present invention, a cellulose-containing raw material is milled by means of a vibration mill filled with rods, whereby cellulose contained in the raw material can be efficiently decrystallized, which is preferred. Examples of the vibration mill using rods as grinding media therefor include a vibration mill available from Chuo Kakohki Co., Ltd., a small-size vibration rod mill "1045 Model" available from Yoshida Seisakusho Co., Ltd., a vibration cup mill "P-9 Model" available from Fritsch Inc. of Germany, and a small-size vibration mill "NB-O Type" available from Nitto Kagaku Co., Ltd. The treating method used in these vibration mills may be either a batch method or a continuous method. The rods to be filled in the vibration mill are bar-like grinding media, and preferably, each rod has a sectional shape such as a polygonal shape; e.g., a square shape or a hexagonal shape, a circular shape, an elliptical shape, etc. The rods to be filled in the vibration mill each have an outer diameter of preferably 0.5 to 200 mm, more preferably 1 to 100 mm, even more preferably 5 to 50 mm. The length of the respective rods is not particularly limited so long as the rods are shorter than the length of the container of the mill. When the size of the rods lies within the above specified range, desired grinding force can be attained, and the cellulose can be efficiently decrystallized without contamination of the cellulose powder which would otherwise be caused by inclusion of fragments of the rods thereinto.

The filling ratio of media such as balls and rods in the mill varies depending upon the kind of vibration mill used, and is preferably 10 to 97%, more preferably 15 to 95%. When the filling ratio falls within the above specified range, the frequency of contact between the cellulose-containing raw material and the media increases, and the grinding efficiency thereof can be enhanced without inhibiting the motion of the grinding media. The "filling ratio" used herein means a ratio of the apparent volume of the media to the volume of the mill.

The treatment time in the mill varies depending upon the kind of the mill as well as the kind, size, and filling ratio of the media such as balls and rods and, therefore, is not particularly limited. From the viewpoint of reducing the crystallinity of the cellulose, the treatment time is preferably 0.01 to 72 hrs, more preferably 0.01 to 50 hrs, even more preferably 0.05 to 20 hrs, still more preferably 0.1 to 10 hrs. The treating temperature in the mill is also not particularly limited, and is preferably 5 to 250°C, more preferably 10 to 200°C from the viewpoint of preventing heat deterioration.

When the above treating method is performed, decrystallized cellulose having cellulose I-type crystallinity of 33% or less can be efficiently produced from the cellulose-containing raw material. In addition, upon the treatment by means of the mill, the cellulose-containing raw material can be treated under dry conditions without allowing the milled material to adhere on the inside of the mill.

The average particle size of the resultant decrystallized cellulose is preferably 25 to 150 $\mu$m, preferably 30 to 100 $\mu$m, the viewpoints of good chemical reactivity and good handling property when the decrystallized cellulose is employed as an industrial raw material. In particular, decrystallized cellulose having an average particle size of 25 $\mu$m or larger can be prevented from forming a so-called "undissolved lump or flour" when coming into contact with a liquid such as water.

In order to efficiently perform milling and decrystallization in the present invention, the bulk density of the cellulose-containing raw material fed to the mill is 100 kg/m$^3$ or more, more preferably 120 kg/m$^3$ or more, still more preferably 150 kg/m$^3$ or more. When the bulk density of the cellulose-containing raw material is 100 kg/m$^3$ or more, the cellulose-containing raw material has an appropriate volume, resulting in improved handling property. Further, in such a case, the amount of the raw material charged into the mill can be increased, resulting in enhanced treating capacity of the mill. On the other hand, the upper limit of the bulk density of the cellulose-containing raw material fed to the mill is 500 kg/m$^3$ or less, more preferably 400 kg/m$^3$ or less, still more preferably 350 kg/m$^3$ or less, from the viewpoints of good handling property and good productivity. From these viewpoints, the bulk density of the cellulose-containing raw material fed to the mill is 100 to 500 kg/m$^3$, more preferably 120 to 400 kg/m$^3$, still more preferably 150 to 350 kg/m$^3$.

The average particle size of the cellulose-containing raw material fed to the mill is 0.01 to 1 mm, from the viewpoint of effective dispersion of a material for milling in the mill. When the cellulose-containing raw material has an average particle size of 1 mm or smaller, the material for milling is fed to the mill can be efficiently dispersed in the mill, and milled into a desired particle size without requiring a prolonged period of time. On the other hand, the lower limit of the average particle size of the cellulose-containing raw material fed to the mill is 0.01 mm or larger, in view of good productivity. From these viewpoints, the average particle size of the cellulose-containing raw material fed to the mill is more preferably 0.01 to 0.7 mm, still more preferably 0.05 to 0.5 mm. Meanwhile, the aforementioned bulk density and average particle size may be measured by the methods described in Examples below.

[Preliminary Treatment before Milling]

In the present invention, the cellulose-containing raw material which is fed to the aforementioned mill is preferably subjected to a preliminary treatment. For example, the cellulose-containing raw material is treated by means of an extruder, whereby the bulk density and the average particle size of the cellulose-containing raw material can be adjusted to fall within preferred ranges.

Before charging the cellulose-containing raw material into the extruder, the material is preferably coarsely milled into chips. The size of the coarsely milled chips is preferably 1 to 50 mm, more preferably 1 to 30 mm. When the coarsely milled chip-like cellulose-containing raw material having a size of from 1 to 50 mm is employed, extruder treatment can be readily conducted in an efficient manner, thereby reducing the load required for milling.

The cellulose-containing raw material may be coarsely milled by means of a shredder or a rotary cutter. When a rotary cutter is employed, the size of the resultant coarsely milled material may be controlled by modifying the mesh size of a screen used therein. The mesh size of the screen is preferably 1 to 50 mm, more preferably 1 to 30 mm. When a screen having a mesh size of 1 mm or more is employed, the resultant coarsely milled material having a suitable bulkiness can be obtained, resulting in enhanced handling property thereof. When a screen having a mesh size of 50 mm or less is employed, the cellulose-containing raw material has a size suitable for the subsequent milling treatment, resulting in reduced load for milling.

When the cellulose-containing raw material (preferably the coarsely milled cellulose-containing raw material) is treated by means of an extruder, a compression shear force may be applied to the cellulose-containing raw material to break the crystal structure of the cellulose and mill the cellulose-containing raw material into a powder.

In the method of mechanically milling the cellulose-containing raw material by applying a compression shear force thereto, if an impact-type mill which has been generally employed in conventional techniques, such as a cutter mill, a hammer mill, a pin mill, etc., is used, the milled material tends to suffer from flocculation and, therefore, very high bulkiness, resulting in poor handling property and deterioration in weight-based treatment capability. On the other hand, the cellulose-containing raw material milled by means of an extruder can exhibit a desired bulkiness and average particle size, resulting in enhanced handling property thereof.

The extruder may be of either a single-screw type or a twin-screw type. From the viewpoint of enhancement in conveying capability, etc., of these apparatuses, the twin-screw extruder is preferably used.

As the twin-screw extruder, there may be used a conventionally known twin-screw extruder in which two screws are rotatably inserted into a cylinder. The rotational directions of the two screws in the twin-screw extruder may be either identical or opposite. From the viewpoint of enhancement in delivering capability, etc., the screws are preferably rotated in the same direction.

The meshing of the screws in the extruder may be of any of a complete meshing type, a partially meshing type, and a de-meshing type. From the viewpoint of enhancement in treating capability, etc., the complete meshing type or the partially meshing type is preferred.

From the viewpoint of applying a strong compression shear force to the cellulose-containing raw material, the extruder is preferably provided with a so-called kneading disk segment in any portion of the respective screws thereof. The kneading disk segment is constituted of a plurality of kneading disks which are continuously arranged in combination while offsetting their positions at a constant phase; for example, at intervals of 90°, and is capable of applying a very strong shear force to the cellulose-containing raw material with rotation of the screws by forcibly passing the raw material through a narrow gap between the kneading disks or between the kneading disk and the cylinder. The screw preferably has such a structure that the kneading disk segments and the screw segments are arranged in an alternate relation to each other. In the twin-screw extruder, the two screws are preferably identical in structure to each other.

Upon the treatment by means of an extruder, it is preferred that the cellulose-containing raw material, preferably the coarsely milled cellulose-containing raw material, is charged into the extruder and continuously treated therein. The shear rate used upon the treatment is preferably 10 sec$^{-1}$ or more, more preferably 20 to 30,000 sec$^{-1}$, even more preferably 50 to 3,000 sec$^{-1}$. When the shear rate is 10 sec$^{-1}$ or more, milling the cellulose-containing raw material proceeds effectively. The other treatment conditions are not particularly limited. The treatment temperature is preferably from 5 to 200°C.

The number of passes of the cellulose-containing raw material through the extruder to attain a sufficient effect may be only one (pass). From the viewpoint of lowering the crystallinity and polymerization degree of the cellulose-containing raw material, if one-pass treatment is unsatisfactory, 2 or more passes are preferably conducted. Also, in view of good productivity, the number of passes of the cellulose-containing raw material through the extruder is preferably 1 to 10 (passes). When the passes are repeated through the extruder, the coarse particles contained in the raw material are milled, thereby obtaining a powdery cellulose-containing raw material having small variation in particle size. When 2 or more passes are conducted, a plurality of the extruders may be arranged in series, in view of high production capacity.

[Saccharification by use of Enzyme such as Cellulase]

Since the decrystallized cellulose produced through the aforementioned treatment has low crystallinity, the cellulose can readily form, through an enzymatic treatment by use of a cellulase, a mixture of glucose and oligosaccharides such as cellobiose or cellotriose at high efficiency. The saccharification is preferably performed to form monosaccharides, in consideration that the saccharification product is subjected to ethanol fermentation or lactic fermentation after saccharification. As used herein, the term "cellulase" refers to enzymes which hydrolyze a glycoside bond of β-1,4-glucan of cellulose and collectively refers to enzymes including endoglucanase, exoglucanase, cellobiohydrase, and β-glucosidase. When a hemicellulase such as xylanase is caused to act in combination with cellulase, saccharification efficiency can be enhanced.

No particular limitation is imposed on the cellulase and hemicellulase employed in the saccharification, and commercially products of cellulase and those derived from animals, plants, and microorganisms may be employed. Examples of the cellulase include cellulase products derived from Trichoderma reesei such as Celluclast 1.5L (product of Novozymes); cellulase derived from a Bacillus sp. KSM-N145 strain (FERM P-19727); cellulase derived from strains of Bacillus sp. KSM-N252 (FERM P-17474), Bacillus sp. KSM-N115 (FERM P-19726), Bacillus sp. KSM-N440 (FERM P-19728), Bacillus sp. KSM-N659 (FERM P-19730), etc.; cellulase mixtures derived from Trichoderma viride, Aspergillus acleatus, Clostridium thermocellum, Clostridium stercorarium, Clostridium josui, Cellulomonas fimi, Acremonium cellulolyticus, Irpex lacteus, Aspergillus niger, and Humicola insolens; and a heat-resistant cellulase derived from Pyrococcus horikoshii. Among them, cellulases derived from Trichoderma reesei, Trichoderma viride, or Humicola insolens (e.g., Celluclast 1.5L (product of Novozymes), TP-60 (product of Meiji Seika Kaisha, Ltd.), or Ultraflo L (product of Novozymes) are preferably used, to thereby efficiently produce saccharide.

Examples of the hemicellulase include a xylanase derived from Bacillus sp. KSM-N546 (FERM P-19729); xylanases derived from Aspergillus niger, Trichoderma viride, Humicola insolens, and Bacillus alcalophilus; and xylanases derived from the genus Thermomyces, Aureobasidium, Streptomyces, Clostridium, Thermotoga, Thermoascus, Caldocellum, and Thermomonospora. Alternatively, an enzyme contained in the aforementioned cellulase mixtures and having a hemicellulase activity may also be employed.

These enzymes may be used singly and, more preferably, the enzymes are used in combination for attaining more efficient production of saccharides. Through addition of a specific cellulase such as β-glucosidase to these enzymes, the efficiency of saccharide production can be enhanced. Examples of the β-glucosidase added to these enzymes include an enzyme derived from Aspergillus niger (e.g., Novozyme 188 (product of Novozymes) or β-glucosidase (product of Megazyme)) and enzymes derived from Trichoderma reesei and Penicillium emersonii.

Reaction conditions under which cellulase-mediated saccharification of the decrystallized cellulose produced through the aforementioned treatment is performed may be appropriately selected, depending on the crystallinity of the decrystallized cellulose obtained through the preliminary treatment and the enzyme used. For example, when Celluclast 1.5L (product of Novozymes) is employed as the cellulase, and a cellulose derived from pulp and having crystallinity of 0% are employed as a substrate, Celluclast 1.5L is added to a 0.5 to 20% (w/v) suspension of the substrate so as to adjust the enzyme concentration of 0.001 to 15% (v/v) (0.00017 to 2.5% as protein), and the enzymatic reaction is performed in a buffer in which pH is adjusted to 2 to 10 (the pH is preferably appropriately selected depending on the type of the enzyme employed and pH is preferably 3 to 7, particularly preferably near 5 in the case of Celluclast 1.5L), at a reaction temperature of 10 to 90°C (the temperature is preferably appropriately selected depending on the type of the enzyme employed and the reaction is preferably performed at 20 to 70°C, particularly preferably about 50°C in the case of Celluclast 1.5L), for 30 minutes to 5 days, more preferably 0.5 to 3 days, to thereby produce saccharide.

EXAMPLES

The average particle size, bulk density, and X-ray diffraction intensity of the decrystallized cellulose or cellulose-containing raw material, and the cellulose content of the raw material were measured through the following methods. Saccharification of celluloses such as decrystallized cellulose was performed under the following conditions.

(1) Measurement of Average Particle Size

The average particle size was measured by means of a laser diffraction/scattering-type particle size distribution measuring device "LA-920" available from Horiba, Ltd. Upon measurement, a sample was subjected to an ultrasonic treatment for 1 min prior to measuring the particle size thereof, and then by using water as a dispersing medium, the average diameter of the sample was measured at 25°C.

(2) Measurement of Bulk Density

The bulk density was measured using a "Powder Tester" available from Hosokawa Micron Corporation. Upon measurement, a sample was caused to fall through a chute on a screen being vibrated, and received in a standard container (having a capacity of 100 mL) to measure the weight of the sample in the container and calculate the bulk density thereof from the measured value.

(3) Measurement of X-ray Diffraction Intensities

The cellulose I-type crystallinity of a sample was calculated from X-ray diffraction intensities thereof which were measured under the following conditions by means of a "Rigaku RINT 2500VC X-RAY diffractometer" available from Rigaku Corporation, according to the above calculation formula (1).

Measuring Conditions:

X-ray source: Cu/K$\alpha$-radiation; tube voltage: 40 kV; tube current: 120 mA; and measuring range: 2θ = 5 to 45°. The sample to be measured was prepared through compressing to form pellets each having an area of 320 mm$^2$ and a thickness of 1 mm. X-ray scanning speed was 10°/min.

(4) Measurement of Water Content

The water content was measured at 150°C by means of an infrared moisture meter "FD-610" available from Kett Electric Laboratory.

(5) Measurement of Cellulose Content

Each of the milled cellulose-containing raw material samples was subjected to Soxhlet extraction with an ethanol-benzene solvent mixture (1:1) for 6 hours and further with ethanol for 4 hours. The extracted sample was dried in vacuum at 60°C. To the dried sample (2.5 g), water (150 mL), sodium chlorite (1.0 g), and acetic acid (0.2 mL) were added, and the mixture was heated at 70 to 80°C for one hour. Subsequently, addition of sodium chlorite and acetic acid and heating were repeated 3 to 4 times until the color of the sample was removed. The resultant white residue was filtered through a glass filter (1G-3), followed by washing sequentially with cold water and acetone. The product was dried at 105°C until the weight thereof reached a constant value, then the weight of residue was measured. The cellulose content was calculated on the basis of the following formula:

$$\text{Cellulose content (wt\%)} = [\text{weight of residue (g)/amount of sample collected (g)}] \times 100$$

(6) Saccharification reaction

Saccharification reaction in the presence of an enzyme was performed under the following conditions. Specifically, an appropriate amount of a substrate (cellulose powder or decrystallized cellulose) was suspended in an enzymatic reaction mixture (3 mL) (containing 100mM citrate buffer (pH: 5.0) and 30 $\mu$g/mL tetracycline (antiseptic) and placed in a capped screw tube (No.3, $\phi$: 21 × 45 mm, product of Maruemu Corporation) in Examples 2-1 and 2-2 and Comparative Example 2-1 or in a capped screw tube (No.5, $\phi$: 27 × 55 mm, product of Maruemu Corporation) in the other Examples and Comparative Examples). Then, an appropriate amount of an enzyme was added to the suspension, and the mixture was stirred with shaking at 50°C by means of a thermostat shaker (Model: BR-15CF, product of TAITEC Corporation) at 150 rpm so that reaction was performed for 6 to 75 hours. After completion of reaction, the reaction mixture was separated into a precipitate and a supernatant through centrifugation (17,000×g, 5 min). The weight of the dried precipitate and the amount of saccharide or reducing saccharide released in the supernatant were quantitatively determined through the phenol-sulfuric acid method, DNS method, or HPLC method, described below. As a control, an unreacted enzymatic reaction mixture was analyzed in the same manner.

(7) Quantitative Determination of Saccharide through Phenol-Sulfuric Acid Method (Text of Bioengineering Experiment, Baifu-kan)

Each supernatant (0.1 mL) was appropriately diluted with ion-exchange water, and 5% (w/w) phenol solution (0.1 mL) was added to the diluted supernatant, followed by mixing. Sulfuric acid (0.5 mL) was further added thereto, followed by sufficiently mixing. The resultant mixture was allowed to stand at room temperature for 20 minutes, and analyzed through colorimetry at wavelength of 490 nm. The total saccharide amount contained in the supernatant was calculated by a calibration curve obtained by use of glucose as a standard saccharide.

(8) Quantitative Determination of Saccharide through DNS Method ("Determination of reducing sugar," Experimental Biochemistry, Gakkai Shuppan Center)

An appropriate amount of each supernatant was added to a DNS solution (1 mL) (0.5% 3,5-dinitrosalicylic acid, 30% sodium potassium tartrate tetrahydrate, and 1.6% sodium hydroxide), and the mixture was heated at 100°C for 5 minutes for color development. After cooling, the mixture was analyzed through colorimetry at wavelength of 535 nm. The amount of reducing saccharide contained in the supernatant was calculated by a calibration curve obtained by use of glucose as a standard saccharide. Note that, since a certain type of the formed saccharides exhibits a color development intensity which is not equal to that of glucose, the determined reducing saccharide amount may differ from the value determined through HPLC.

(9) Quantitative Determination of Saccharide through HPLC Method

The determination was performed by means of a DX500 Chromatography System (product of Dionex Corporation) (column: CarboPac PA1 (Dionex Corporation, $4 \times 250$ mm) and detector: ED40 pulsed amperometry detector). The following eluents were employed: (A) 100mM sodium hydroxide solution, (B) 100mM sodium hydroxide solution containing 1M sodium acetate, and (C) ultrapure water. Sugar analysis was performed under linear gradient conditions: A 10%-C 90% (at injection) and A 95%-B 5% (0 to 15 min). As standards, 0.01% (w/v) glucose (product of Wako Pure Chemical Industries, Ltd.), xylose (Wako Pure Chemical Industries, Ltd.), xylobiose (Wako Pure Chemical Industries, Ltd.), and cellobiose (product of Seikagaku Corporation).

(10) Quantitative Determination of Protein

A DC Protein Assay Kit (product of Bio-Rad Laboratories) was employed, and weight of protein was calculated by a calibration curve obtained by use of bovine serum albumin as a standard protein.

PRODUCTION EXAMPLE 1-1

[Shredder Treatment]

A sheet-like wood pulp as a cellulose-containing raw material ["Blue Bear Ultra Ether" available from Borregaard, size: 800 mm $\times$ 600 mm $\times$ 1.5 mm; crystallinity: 81%; cellulose content: 96% by weight; water content: 7% by weight] was cut by means of a shredder "MSX2000-IVP440F" available from Meikoshokai Co., Ltd., to prepare chipped pulp heaving a size of about 10 mm $\times$ 5 mm $\times$ 1.5 mm.

[Vibration Mill Treatment]

The resultant chipped pulp (100 g) was charged into a vibration mill "MB-1" available from Chuo Kakohki Co., Ltd., (total container capacity: 3.5 L), and treated therein at a vibration amplitude of 8 mm and a circular rotation speed of 1,200 cpm for 3 hr under such conditions that 16 stainless steel rods each having a circular shape in section, a diameter of 25 mm and a length of 218 mm were placed in the vibration mill (filling ratio: 49%). The decrystallized cellulose obtained after the treatment using the vibration mill had an average particle size of 80 $\mu$m. Also, the temperature of the resultant decrystallized cellulose was as high as 85°C owing to heat generated upon the treatment. After completion of the treatment, no pulp adhered on the inner wall surface and bottom of the vibration mill was observed. The thus-obtained decrystallized cellulose was taken out of the vibration mill and subjected to measurements of an average particle size and X-ray diffraction intensity thereof. The crystallinity of the decrystallized cellulose was calculated from the measured X-ray-diffraction intensity. The results are shown in Table 1-1.

PRODUCTION EXAMPLE 1-2

[Extruder Treatment]

The chipped pulp, which had been obtained from the same sheet-like wood pulp through the same method both as employed in Production Example 1-1, was charged into a twin-screw extruder "EA-20" available from Suehiro EPM Corporation, at a feed rate of 2 kg/h, and passed therethrough one time (one pass) at a shear rate of 660 sec$^{-1}$ and a screw rotating speed of 300 rpm while flowing cooling water from outside therethrough. Meanwhile, the twin-screw extruder used was of a complete meshing and unidirectional rotation type in which the screws arranged in two rows were each provided with a screw segment having a screw diameter of 40 mm and a kneading disk segment constituted of a combination of 12 blocks of kneading disks offset from each other at intervals of 90°, and the two screws had the same construction. In addition, the temperature in the twin-screw extruder was raised to from 30 to 70°C owing to heat generated upon the treatment.

As a result, it was confirmed that the pulp obtained after the extruder treatment had an average particle size of 121 $\mu$m and a bulk density of 254 kg/m$^3$.

[Vibration Mill Treatment]

The pulp obtained through the extruder treatment was subjected to vibration milling in a manner similar to that employed in Production Example 1-1, to thereby produce decrystallized cellulose. After completion of shredding, no pulp adhered on the inner wall surface and bottom of the vibration mill was observed. The results are shown in Table 1-1.

PRODUCTION EXAMPLE 1-3

The same procedure as described in Production Example 1-2 was repeated except for filling 13 stainless steel rods each having a circular shape in section, a diameter of 30 mm and a length of 218 mm into the vibration mill (filling ratio: 57%) and operating the vibration mill for 1 hr, thereby obtaining decrystallized cellulose. The results are shown in Table 1-1.

PRODUCTION EXAMPLE 1-4

The same procedure as described in Production Example 1-2 was repeated except for filling the 14 stainless steel rods into the vibration mill (filling ratio: 62%), thereby obtaining decrystallized cellulose. The results are shown in Table 1-1.

PRODUCTION EXAMPLE 1-5

The same procedure as described in Production Example 1-2 was repeated except for filling the 8 stainless steel rods each having a diameter of 36 mm and a length of 218 mm into the vibration mill (filling ratio: 51%) and operating the vibration mill for 1 hr, thereby obtaining decrystallized cellulose. The results are shown in Table 1-1.

PRODUCTION EXAMPLE 1-6

The same procedure as described in Production Example 1-2 was repeated except for filling the 11 stainless steel rods each having a diameter of 30 mm and a length of 218 mm into the vibration mill (filling ratio: 48%) and operating the vibration mill for 3 hr, thereby obtaining decrystallized cellulose. The results are shown in Table 1-1.

[Table 1]

Table 1-1

| | | | Production Example | | | | | |
|---|---|---|---|---|---|---|---|---|
| | | | 1-1 | 1-2 | 1-3 | 1-4 | 1-5 | 1-6 |
| Shredding | | | yes | yes | yes | yes | yes | yes |
| Twin-screw extruder treatment | Treatment | | no | yes | yes | yes | yes | yes |
| | Shear rate (sec$^{-1}$) | | | 660 | 660 | 660 | 660 | 660 |
| | Av. particle size ($\mu$m) | | | 121 [*1] | 121 [*1] | 121 [*1] | 121[*1] | 121 [*1] |
| | Bulk density (kg/m$^3$) | | | 254[*1] | 254 [*1] | 254 [*1] | 254 [*1] | 254 [*1] |
| Vibration mill treatment | Shape of grinding media in container | | rods | rods | rods | rods | rods | rods |
| | Rod diameter (mm) | | 25 | 25 | 30 | 25 | 36 | 30 |
| | Number of rods | | 16 | 16 | 13 | 14 | 8 | 11 |
| | Amount of pulp charged (g) | | 100 | 100 | 100 | 100 | 100 | 100 |
| | Treatment time (h) | | 2 | 2 | 1 | 2 | 1 | 3 |

(continued)

| | | Production Example | | | | | |
|---|---|---|---|---|---|---|---|
| | | 1-1 | 1-2 | 1-3 | 1-4 | 1-5 | 1-6 |
| Evaluation | Cellulose I-type Crystallinity (%) | 0 | 0 | 0 | 0 | 0 | 0 |
| | Occurrence of deposit after milling *2 | no | no | no | no | no | no |
| | Av. particle size of decrystallized cellulose ($\mu$m) *3 | 80 | 57 | 68 | 88 | 55 | 57 |

*1: average particle size or bulk density or pulp after the twin-screw extruder treatment
*2: Presence or absence of pulp adhered in the vibration mill after the vibration mill treatment
*3: Av. particle size of decrystallized cellulose obtained after the vibration mill treatment

COMPARATIVE PRODUCTION EXAMPLE 1-1

The same shredder treatment as described in Production Example 1-1 was conducted to obtain chipped pulp. Next, the resultant chipped pulp (100 g) was charged into a tumbling mill "Pot Mill ANZ-51S" available from Nitto Kagaku Co., Ltd., (container capacity: 1.0 L; 10 mm$\phi$ zirconia balls filled: 1.8 kg; filling ratio: 53%), and treated by means of the tumbling mill at a rotating speed of 100 rpm for 48 hr. It was confirmed that the pulp was not powdered, and still kept substantially in a chipped state. The crystallinity of the obtained pulp was calculated from the measured X-ray diffraction intensities thereof by the method described above. The results are shown in Table 1-2.

COMPARATIVE PRODUCTION EXAMPLE 1-2

The same shredder treatment as described in Production Example 1-1 was conducted to obtain chipped pulp. Next, the resultant chipped pulp (500 g) was charged into a cutter mill "POWER MILL P-02S Model" available from Dalton Co., Ltd., and treated therein at a rotating speed of 3,000 rpm for 0.5 hr. As a result, the resultant milled product was flocculated, thereby failing to obtain decrystallized cellulose. The results are shown in Table 1-2.

COMPARATIVE PRODUCTION EXAMPLE 1-3

The same shredder treatment as described in Production Example 1-1 was conducted to obtain chipped pulp. Next, the resultant chipped pulp (500 g) was charged into a hammer mill "SAMPLE-MILL" available from Dalton Co., Ltd., and treated therein at a rotating speed of 13,500 rpm for 0.5 hr. As a result, the resultant milled product was flocculated, thereby failing to obtain decrystallized cellulose. The results are shown in Table 1-2.

COMPARATIVE PRODUCTION EXAMPLE 1-4

The same shredder treatment as described in Production Example 1-1 was conducted to obtain chipped pulp. Next, the resultant chipped pulp (500 g) was charged into a pin mill "KOLLOPLEX" available from Hosokawa Micron Corporation, and treated therein at a rotating speed of 13,000 rpm for 0.25 hr. As a result, the resultant milled product was flocculated, thereby failing to obtain decrystallized cellulose. The results are shown in Table 1-2.

COMPARATIVE PRODUCTION EXAMPLE 1-5

The same procedure as described in Production Example 1-1 was repeated, to thereby produce chipped pulp. Then, in a manner similar to that of Production Example 1-2, the chipped pulp was subjected to the twin-screw extruder treatment, but no mill treatment was conducted, thereby obtaining powdered pulp. Through the aforementioned analysis methods, the resultant powdered pulp was subjected to measurements of an average particle size and X-ray diffraction intensity thereof. The crystallinity of the obtained product was calculated from the measured X-ray diffraction intensity. The results are shown in Table 1-2.
[Table 2]

Table 1-2

| | | Comparative (Production Examples | | | | |
|---|---|---|---|---|---|---|
| | | 1-1 | 1-2 | 1-3 | 1-4 | 1-5 |
| Shredding | | yes | yes | yes | yes | yes |
| Twin-screw extruder treatment | Treatment | no | no | no | no | yes |
| Mill treatment | Treatment | yes | yes | yes | yes | no |
| | Kind of mill | Tumbling mill | Cutter mill | Hammer mill | Pin mill | - |
| | Kind of balls | $\phi$10 mm zirconia | - | - | - | - |
| | Amount of pulp charged (g) | 100 | 500 | 500 | 500 | - |
| | Treatment time (hr) | 48 | 0.5 | 0.5 | 0.25 | - |
| Evaluation | Cellulose I-type Crystallinity (%) | 73 | 78 | 74 | 75 | 76 |
| | Av. particle size of pulp ($\mu$m)[*1] | (Almost chip-like) | (Floccu -lated) | (Floccu -lated) | (Floccu -lated) | 156 |
| *1: Average particle size of pulp after the mill treatment | | | | | | |

PRODUCTION EXAMPLES 1-7 to 1-10

Cellulose-containing materials (wood-free paper (Production Example 1-7, cellulose content: 83% by weight and water content: 5.7% by weight), corrugated cardboard (Production Example 1-8, cellulose content: 84% by weight and water content 7.2% by weight), newspaper (Production Example 1-9, cellulose content: 83% by weight and water content: 7.7% by weight), and chaff (Production Example 1-10, cellulose content: 60% by weight and water content: 13.6% by weight) were subjected to the shredder treatment through the method and under the conditions described in Production Example 1-1 and then to the extruder treatment through the method and under the conditions described Production Example 1-2. The properties of the cellulose-containing materials obtained after the extruder treatment are as follows: (Production Example 1-7) wood-free paper, average particle size of 71 $\mu$m/bulk density of 274 kg/m$^3$; (Production Example 1-8) corrugated cardboard, average particle size of 93 $\mu$m/bulk density of 216 kg/m$^3$; (Production Example 1-9) newspaper, average particle size of 61 $\mu$m/bulk density of 303 kg/m$^3$; and (Production Example 1-10) chaff, average particle size of 85 $\mu$m/bulk density of 380 kg/m$^3$.
These materials were further subjected to the vibration mill treatment through the method and under the conditions described in Production Example 1-6, to thereby produce decrystallized celluloses. The properties thereof are as follows: (Production Example 1-7) wood-free paper, crystallinity of 0%/average particle size of 42 $\mu$m, (Production Example 1-8) corrugated cardboard, crystallinity of 0%/average particle size of 48 $\mu$m, (Production Example 1-9) newspaper, crystallinity of 0%/average particle size of 55 $\mu$m, and (Production Example 1-10) chaff, crystallinity of 0%/average particle size of 48 $\mu$m).

PRODUCTION EXAMPLES 1-11 to 1-12

[Coarse mill treatment of pruned-off branches]

As cellulose-containing raw materials, a mixture of rod-shaped, pruned-off branches of roadside trees (Prunus yedoensis, Quercus phillyraeoides, camphor tree, Quercus acutissima, and Campsis grandiflora) (Production Example 1-11) ($\phi$: 10 mm $\times$ 300 mm, cellulose content: 67% by weight, and water content: 12% by weight), and rod-shaped, pruned-off branches of tangerine trees (Production Example 1-12) ($\phi$: 10 mm $\times$ 500 mm, cellulose content: 64% by weight, and water content: 22% by weight) were milled by means of a plastic mill (Model: JC-2, product of Morita Seiki Kogyo Co., Ltd.), to thereby produce chips (about 2 mm $\times$ 3 mm $\times$ 1 mm) of the materials. Subsequently, the produced roadside tree chips and chips of pruned-off branches of a tangerine tree were dried by means of a drier to a water content of 2.3% by weight and 3.5% by weight, respectively. The chips of pruned-off branches of tangerine trees were found to have a bulk density of 224 kg/m$^3$.

[Vibration mill treatment of pruned-off branches]

The thus-produced chips of pruned-off branches were further subjected to the vibration mill treatment through the method and under the conditions described in Production Example 1-6, to thereby produce decrystallized celluloses. The properties thereof are as follows: (Production Example 1-11) branches of roadside trees, crystallinity of 0%/average particle size of 49 $\mu$m, and (Production Example 1-12) tangerine trees, crystallinity of 0%/average particle size of 44 $\mu$m.

COMPARATIVE PRODUCTION EXAMPLES 1-6 to 1-11

The cellulose-containing raw materials of Production Examples 1-7 to 1-10 were subjected to the shredder treatment and the extruder treatment in the same manner. The roadside tree pruned-off branches of Production Example 1-11 and the tangerine trees pruned-off branches of Production Example 1-12 were also subjected to the coarse mill treatment by means of a plastic mill in the same manner, but not to the vibration mill treatment. Thus, powdered and chipped cellulose products were yielded. The properties thereof are as follows: (Comparative Production Example 1-6) wood-free paper, crystallinity of 71%/average particle size of 71 $\mu$m; (Comparative Production Example 1-7) corrugated cardboard, crystallinity of 71%/average particle size of 93 $\mu$m; (Comparative Production Example 1-8) newspaper, crystallinity 56%/average particle size of 61 $\mu$m; (Comparative Production Example 1-9) chaff, crystallinity of 47%/average particle size of 85 $\mu$m; (Comparative Production Example 1-10) roadside tree pruned-off branches, crystallinity of 51%/dimensions of 2 mm $\times$ 3 mm $\times$ 1 mm; and (Comparative Production Example 1-11: tangerine trees pruned-off branches, crystallinity of 46%/dimensions of 2 mm $\times$ 3 mm $\times$ 1 mm.

EXAMPLES 1-1 AND COMPARATIVE EXAMPLE 1-1

In Example 1-1, the decrystallized cellulose (crystallinity: 0%, particle size: 57 $\mu$m) prepared through the extruder treatment and the vibration mill treatment employing a rod medium in Production Example 1-6 was subjected to saccharification reaction by use of a cellulases standard sample (Celluclast 1.5L, product of Novozymes). Similarly, in Comparative Example 1-1, the powdered pulp (crystallinity: 76%, particle size: 156 $\mu$m) produced through the extruder treatment in Comparative Production Example 1-5 was subjected to the same reaction. The decrystallized cellulose or powdered pulp (0.15 g) was suspended in an enzymatic reaction mixture (3 mL) (containing 100mM citrate buffer (pH: 5.0), 3% (v/v) Celluclast 1.5L (0.5% as protein), and 30 $\mu$g/mL tetracycline), and the mixture was caused to react at 50°C for 18 hours, 26 hours, and 42 hours, during which the mixture was stirred with shaking. After completion of reaction, the reaction mixture was separated into a precipitate and a supernatant through centrifugation. The amount of reducing saccharide (as glucose) released to the supernatant was quantitatively determined through the DNS method. The results are shown in Table 1-3.

[Table 3]

Table 1-3

| | Sample | Crystallinity (%) | Amount of reducing saccharide released in supernatant (g/L) | | | |
|---|---|---|---|---|---|---|
| | | | Reaction time (hr) | | | |
| | | | 0 (unreacted) | 18 | 26 | 42 |
| Ex. 1-1 | Decrystallized cellulose | 0 | 0 | 39.7 | 40.8 | 46.0 |
| Comp. Ex. 1-1 | Powdered pulp | 76 | 0 | 21.7 | 24.8 | 29.9 |

EXAMPLES 1-2 to 1-4 and COMPARATIVE EXAMPLES 1-2 to 1-4

In Examples 1-2 to 1-4, decrystallized celluloses were prepared under the aforementioned conditions of Production Example 1-6 (Examples 1-2 to 1-4: crystallinity of 0%/average particle size of 57 $\mu$m). In Comparative Examples 1-2 to 1-4, a powdered pulp product (Comparative Examples 1-2 to 1-4: crystallinity of 76%/average particle size of 156 $\mu$m) prepared in Comparative Production Example 1-5 was employed. Each of the samples was subjected to saccharification reaction by use of a cellulases standard sample (Celluclast 1.5L, product of Novozymes). Each of the decrystallized celluloses and the powdered pulp product (0.15 g) was suspended in an enzymatic reaction mixture (3 mL), and the mixture was caused to react at 50°C for 6 hours, 24 hours, 48 hours, and 72 hours, during which the mixture was stirred with shaking. The following enzymatic reaction mixtures were employed: (Example 1-2 and Comparative Example 1-2) 100mM citrate buffer (pH: 5.0), 3% (v/v) Celluclast 1.5L (0.5% as protein), and 30 μg/mL tetracycline); (Example 1-3 and Comparative Example 1-3) 100mM citrate buffer (pH: 5.0), 1.5% (v/v) Celluclast 1.5L (0.25% as protein), and 30 μg/mL tetracycline); and (Example 1-4 and Comparative Example 1-4) 100mM citrate buffer (pH: 5.0), 0.6% (v/v) Celluclast 1.5L (0.1% as protein), and 30 μg/mL tetracycline). After completion of reaction, the reaction mixture was separated into a precipitate and a supernatant through centrifugation. The amount of reducing saccharide (as glucose) released to the supernatant was quantitatively determined through the DNS method. The results are shown in Table 1-4.

[Table 4]

Table 1-4

| | Sample | Crystallinity (%) | Amount of redudng saccharide released in supernatant (g/L) | | | |
|---|---|---|---|---|---|---|
| | | | Reaction time (hr) | | | |
| | | | 6 | 24 | 48 | 72 |
| Ex. 1-2 | Decrystallized cellulose | 0 | 25.8 | 45.8 | 47.7 | 58.4 |
| Comp. Ex. 1-2 | Powdered pulp | 76 | 13.4 | 22.5 | 31.1 | 34.3 |
| Ex. 1-3 | Decrystallized cellulose | 0 | 18.7 | 36.0 | 40.3 | 45.7 |
| Comp. Ex. 1-3 | Powdered pulp | 76 | 8.8 | 18.4 | 23.1 | 28.1 |
| Ex. 1-4 | Decrystallized cellulose | 0 | 14.3 | 23.3 | 32.9 | 40.3 |
| Comp. Ex. 1-4 | Powdered pulp | 76 | 6.1 | 15.0 | 21.0 | 20.8 |

EXAMPLE 1-5

The supernatant of the saccharification mixture (reaction time: 72 hours) obtained in Example 1-2 was analyzed in the following manner by means of a DX500 Chromatography System (Dionex Corporation) (column: CarboPac PA1 (Dionex Corporation, 4 × 250 mm) and detector: ED40 pulsed amperometry detector). The following eluents were employed: (A) 100mM sodium hydroxide solution, (B) 100mM sodium hydroxide solution containing 1M sodium acetate, and (C) ultrapure water.

Sugar analysis was performed under linear gradient conditions: A 10%-C 90% (at injection) and A 95%-B 5% (0 to 15 min). As standards, 0.01% (w/v) glucose (product of Wako Pure Chemical Industries, Ltd.), xylose (Wako Pure Chemical Industries, Ltd.), xylobiose (Wako Pure Chemical Industries, Ltd.), and cellobiose (product of Seikagaku Corporation). The retention times of the standards are as follows: glucose (about 5.5 min), xylose (about 6.5 min), xylobiose (about 14 min), and cellobiose (about 14.5 min). Each supernatant of the saccharification mixture was 100-fold diluted, and an aliquot (10 μL) was injected. The results are shown in Table 1-5.

[Table 5]

Table 1-5

| | Sample | Amount of saccharide released in supernatant (g/L) | | | |
|---|---|---|---|---|---|
| | | Reaction time (72 hr) | | | |
| | | glucose | xylose | xylobiose | cellobiose |
| Ex. 1-5 | Decrystallized cellulose | 43 | 3.9 | 3.5 | 3.8 |

EXAMPLES 1-6 to 1-11

The decrystallized cellulose (crystallinity of 0%/average particle size of 57 μm) prepared under the conditions employed in Production Example 1-6 was to saccharification reaction by use of a cellulases standard sample (TP-60, product of Meiji Seika Kaisha, Ltd., 650 mg-protein/g) (Examples 1-6 to 1-8) or (Ultraflo L, product of Novozymes, 50 mg-protein/mL) (Examples 1-9 to 1-11). Each of the decrystallized celluloses (0.15 g) was suspended in an enzymatic reaction mixture (3 mL), and the mixture was caused to react at 50°C for 24 hours, 48 hours, and 72 hours, during which the mixture was stirred with shaking. The following enzymatic reaction mixtures were employed: (Example 1-6) 100mM citrate buffer (pH: 5.0), 0.83% (w/v) TP-60 (0.54% as protein), and 30 μg/mL tetracycline); (Example 1-7) 100mM citrate buffer (pH: 5.0), 0.42% (w/v) TP-60 (0.28% as protein), and 30 μg/mL tetracycline); (Example 1-8) 100mM citrate buffer (pH: 5.0), 0.17% (w/v) TP-60 (0.11% as protein), and 30 μg/mL tetracycline); (Example 1-9) 100mM citrate buffer (pH: 5.0), 10% (v/v) Ultraflo L (0.5% as protein), and 30 μg/mL tetracycline); (Example 1-10) 100mM citrate buffer (pH: 5.0), 5% (v/v) Ultraflo L (0.25% as protein), and 30 μg/mL tetracycline);and (Example 1-11) 100mm citrate buffer (pH: 5.0), 2% (v/v) Ultraflo L (0.1% as protein), and 30 μg/mL tetracycline). After completion of reaction, the reaction mixture was separated into a precipitate and a supernatant through centrifugation. The amount of reducing saccharide (as glucose) released to the supernatant was quantitatively determined through the DNS method. The results are shown in Table 1-6.

[Table 6]

Table 1-6

| | Enzyme product | Amount of reducing saccharide released in supernatant (g/L) | | |
| | | Reaction time (hr) | | |
| | | 24 | 48 | 72 |
| Ex. 1-6 | TP-60 | 39.4 | 45.6 | 48.8 |
| Ex. 1-7 | TP-60 | 35.9 | 41.7 | 44.2 |
| Ex. 1-8 | TP-60 | 26.0 | 35.2 | 37.7 |
| Ex. 1-9 | Ultraflo L | 24.3 | 37.0 | 36.0 |
| Ex. 1-10 | Ultraflo L | 20.8 | 27.3 | 32.1 |
| Ex. 1-11 | Ultraflo L | 20.4 | 19.6 | 23.7 |

EXAMPLES 1-12 and 1-13

In Examples 1-12 and 1-13, the decrystallized cellulose (crystallinity of 0%/average particle size of 57 μm) prepared under the conditions employed in Production Example 1-6 was to saccharification reaction by use of a cellulases standard sample (Celluclast 1.5L, products of Novozymes) (Example 1-12) or (Celluclast 1.5L and Novozyme 188 (β-glucosidase) products of Novozymes) (Example 1-13). Each of the decrystallized celluloses (0.15 g) was suspended in an enzymatic reaction mixture (3 mL), and the mixture was caused to react at 50°C for 6 hours, 24 hours, 48 hours, and 75 hours, during which the mixture was stirred with shaking.
The following enzymatic reaction mixtures were employed:
(Example 1-12) 100mM citrate buffer (pH: 5.0), 0.6% (v/v) Celluclast 1.5L (0.1% as protein), and 30 μg/mL tetracycline and (Example 1-13) 100mM citrate buffer (pH: 5.0), 0.6% (v/v) Celluclast 1.5L (0.1% as protein), 0.03% (v/v) Novozyme 188 (0.0075% as protein), and 30 μg/mL tetracycline. After completion of reaction, the reaction mixture was separated into a precipitate and a supernatant through centrifugation. The amount of reducing saccharide (as glucose) released to the supernatant was quantitatively determined through the DNS method. The results are shown in Table 1-7.

[Table 7]

Table 1-7

| | Enzyme product | Amount of reducing saccharide released in supernatant (g/L) | | | |
| | | Reaction time (hr) | | | |
| | | 6 | 24 | 48 | 75 |
| Ex. 1-12 | Celluclast 1.5L | 11.1 | 22.0 | 29.0 | 30.5 |
| Ex. 1-13 | Celluclast 1.5L Novozyme 188 | 11.9 | 26.6 | 33.9 | 39.9 |

EXAMPLES 1-14 and 1-15

The supernatants (reaction time: 75 hours) of the saccharification mixtures obtained in Examples 1-12 and 1-13 were analyzed by means of a DX500 Chromatography System (Dionex Corporation) in a manner similar to that of Example 1-5. The results are shown in Table 1-8.

[Table 8]

Table 1-8

| | Enzyme product | Amount of redudng saccharide released in supernatant (g/L) | | | |
| --- | --- | --- | --- | --- | --- |
| | | Reaction time (75 hr) | | | |
| | | glucose | xylose | xylobiose | cellobiose |
| Ex. 1-14 | Celluclast 1.5L | 24.6 | 2.6 | 0 | 17.8 |
| Ex. 1-15 | Celluclast 1.5L Novozyme 188 | 36.7 | 3.2 | 4.8 | 3.0 |

EXAMPLES 1-16 to 1-18 and COMPARATIVE EXAMPLES 1-5 to 1-7

In Examples 1-16 to 1-18, decrystallized celluloses produced in Production Examples 1-7 to 1-9 ((Example 1-16) wood-free paper, crystallinity of 0%/average particle size of 42 $\mu$m, (Example 1-17) corrugated cardboard, crystallinity of 0%/average particle size of 48 $\mu$m, and (Example 1-18) newspaper, crystallinity of 0%/average particle size of 55 $\mu$m) were employed. In Comparative Examples 1-5 to 1-7, powdered celluloses produced in Comparative Production Examples 1-6 to 1-8 ((Comparative Example 1-5) wood-free paper, crystallinity of 71%/average particle size of 71 $\mu$m, (Comparative Example 1-6) corrugated cardboard, crystallinity of 71%/average particle size of 93 $\mu$m, and (Comparative Example 1-7 newspaper, crystallinity of 56%/average particle size of 61 $\mu$m) were employed. In the Examples and Comparative Examples, each of the cellulose products was subjected to saccharification reaction by use of a cellulases standard product (Celluclast 1.5L, product of Novozymes). Each of the decrystallized celluloses and powdered celluloses (0.15 g) was suspended in an enzymatic reaction mixture (3 mL), and the mixture was caused to react at 50°C for 6 hours, 24 hours, 48 hours, and 72 hours, during which the mixture was stirred with shaking. The following enzymatic reaction mixtures were employed: (Examples 1-16 and 1-17 and Comparative Examples 1-5 and 1-6) 100mM citrate buffer (pH: 5.0), 1.5% (v/v) Celluclast 1.5L (0.25% as protein), and 30 $\mu$g/mL tetracycline and (Example 1-18 and Comparative Example 1-7) 100mM citrate buffer (pH: 5.0), 12% (v/v) Celluclast 1.5L (2% as protein), and 30 $\mu$g/mL tetracycline. After completion of reaction, the reaction mixture was separated into a precipitate and a supernatant through centrifugation. The amount of reducing saccharide (as glucose) released to the supernatant was quantitatively determined through the DNS method. The results are shown in Table 1-9.

[Table 9]

Table 1-9

| | Cellulose-containing raw material | Sample | Crystallinity (%) | Amount of reducing saccharide released in supernatant (g/L) | | | |
| --- | --- | --- | --- | --- | --- | --- | --- |
| | | | | Reaction time (hr) | | | |
| | | | | 6 | 24 | 48 | 72 |
| Ex. 1-16 | Wood-free paper | Decrystallized cellulose | 0 | 11.0 | 21.9 | 25.8 | 33.7 |
| Comp. Ex. 1-5 | | Powdered cellulose | 71 | 10.6 | 13.3 | 18.7 | 21.8 |
| Ex. 1-17 | Corrugated cardboard | Decrystallized cellulose | 0 | 15.0 | 23.8 | 27.0 | 31.2 |
| Comp. Ex. 1-6 | | Powdered cellulose | 71 | 8.8 | 12.3 | 13.2 | 13.1 |
| Ex. 1-18 | Newspaper | Decrystallized cellulose | 0 | 21.8 | 23.7 | 26.9 | 30.6 |
| Comp. Ex. 1-7 | | Powdered cellulose | 56 | 12.7 | 9.3 | 11.9 | 13.2 |

EXAMPLES 1-19 to 1-21 and COMPARATIVE EXAMPLES 1-8 to 1-10

In Examples 1-19 to 1-21, decrystallized celluloses ((Example 1-19) chaff, crystallinity of 0%/average particle size of 48 μm, (Example 1-20) pruned-off branches of roadside trees, crystallinity of 0%/average particle size of 49 μm, and (Example 1-21) branches of a tangerin tree, crystallinity of 0%/average particle size 44 μm), produced in Production Examples 1-10 to 1-12,) were employed. In Comparative Examples 1-8 to 1-10, powdered and chipped celluloses ((Comparative Example 1-8) chaff, crystallinity of 47%/average particle size of 85 μm, (Comparative Example 1-9) pruned-off branches of roadside trees, crystallinity of 51%/dimensions of 2 mm × 3 mm × 1 mm, and (Comparative Example 1-10) pruned-off branches of tangerine trees, crystallinity of 46%/dimesions of 2 mm × 3 mm × 1 mm), produced in Comparative Production Examples 1-9 to 1-11), were employed. In the Examples and Comparative Examples, each of the cellulose products was subjected to saccharification reaction by use of a cellulases standard product (Celluclast 1.5L, product of Novozymes). Each of the decrystallized celluloses or powdered celluloses (0.15 g) was suspended in an enzymatic reaction mixture (3 mL), and the mixture was caused to react at 50°C for 3 days, during which the mixture was stirred with shaking. The enzymatic reaction mixture employed was 100mM citrate buffer (pH: 5.0), 3% (v/v) Celluclast 1.5L (0.5% as protein), and 30 μg/mL tetracycline. After completion of reaction, the reaction mixture was separated into a precipitate and a supernatant through centrifugation. The amount of reducing saccharide (as glucose) released to the supernatant was quantitatively determined through the DNS method. The results are shown in Table 1-10.
[Table 10]

Table 1-10

| | Cellulose-containing raw material | Sample | Crystallinity (%) | Amount of reducing saccharide released in supernatant (g/L) |
|---|---|---|---|---|
| | | | | Reaction time (72 hr) |
| Ex. 1-19 | Chaff | Decrystallized cellulose | 0 | 24.4 |
| Comp. Ex. 1-8 | | Powdered cellulose | 47 | 3.1 |
| Ex. 1-20 | Pruned-off branches of roadside trees | Decrystallized cellulose | 0 | 29.0 |
| Comp. Ex. 1-9 | | Chipped cellulose | 51 | 1.8 |
| Ex. 1-21 | Pruned-off branches of tengerine trees | Decrystallized cellulose | 0 | 27.5 |
| Comp. Ex. 1-10 | | Chipped cellulose | 46 | 4.0 |

From Tables 1-1 and 1-2, it was confirmed that the processes for production of the decrystallized celluloses according to Production Examples 1-1 to 1-6 were capable of producing decrystallized cellulose having reduced crystallinity in an efficient manner and were therefore excellent in productivity as compared to those according to Comparative Production Examples 1-1 to 1-5. In addition, from the comparison between Production Example 1-1 and Comparative Production Example 1-1, it was confirmed that the process employing rods as grinding media for the vibration mill according to the present invention was capable of producing such decrystallized cellulose whose crystallinity was reduced to 0%, in an efficient manner. The decrystallized celluloses produced in Production Examples 1-1 to 1-6 were found to have an appropriate average particle size, as compared with those produced in Comparative Production Examples 1-2 to 1-5.
As is clear from Tables 1-3 and 1-4, the process for producing saccharide of the present invention shown in Examples 1-1 to 1-4 exhibited higher productivity as compared with Comparative Examples 1-1 to 1-4. As is clear from Table 1-5, the produced saccharide was mainly formed of glucose. Thus, the process of the present invention is useful for the production of a raw material for producing ethanol or lactic acid through fermentation. As is clear from Table 1-6, the production of saccharide can be efficiently attained regardless of the type of the enzyme product employed in the process. Also, as is clear from Tables 1-7 and 1-8, addition of β-glucosidase to the cellulase product realizes more efficient production of saccharide. As is clear from Tables 1-9 and 1-10, the process for producing saccharide of the present invention is effective for various cellulose materials; i.e., not only for pulp but also for paper materials such as wood-free paper, corrugated cardboard, and newspaper; plant shells such as chaff; and wood materials.

PRODUCTION EXAMPLE 2-1

The same sheet-like wood pulp as employed in Production Example 1-1 was subjected to the same shredder treatment as employed in Production Example 1-1 and to the same extruder treatment in as employed Production Example 1-2. The temperature of the twin-screw extruder employed was elevated to 30 to 70°C by heat generated during the treatment. The pulp after the treatment had an average particle size of 120 μm and a bulk density of 219 kg/m³. Subsequently, the resultant pulp (130 g) was charged into a batch-type agitation tank mill "Sand Grinder" available from Igarashi Kikai Co., Ltd., (container capacity: 800 mL; 5 mmφ zirconia beads filled: 720 g; filling ratio: 25%; diameter of agitation blade: 70 mm). While flowing cooling water through a jacket of the container, the milling treatment was conducted for 2.5 hr at a stirring speed of 2,000 rpm, thereby obtaining decrystallized cellulose.

After completion of the milling treatment, no pulp adhered on the inner wall surface or bottom of the agitation tank mill was observed. The thus-obtained decrystallized cellulose was taken out of the agitation tank mill and passed through a sieve having a mesh size of 75 μm, thereby obtaining 117 g of the decrystallized cellulose as an undersize product (corresponding to 90% by weight on the basis of the material charged). The resultant undersize product was subjected to measurements of an average particle size through the aforementioned method, and the crystallinity thereof was calculated from measured X-ray diffraction intensities. The results are shown in Table 2-1.

PRODUCTION EXAMPLE 2-2

The same procedure as described in Production Example 2-1 was repeated except that a batch-type vibration mill "MB-1" available from Chuo Kakohki Co., Ltd., (container capacity: 2.8 L; 20 mmφ zirconia balls filled: 7.6 kg; filling ratio: 80%) was used in place of the batch-type agitation tank mill, and the pulp (200 g) as the cellulose-containing raw material was charged into the mill and treated therein at a vibration frequency of 20 Hz and a total vibration amplitude of 8 mm for 4 hr, thereby obtaining decrystallized cellulose. After completion of the milling treatment, no pulp adhered on an inner wall surface or a bottom of the vibration mill was observed. The thus-obtained decrystallized cellulose was passed through a sieve having a mesh size of 75 μm, thereby obtaining 142 g of an undersize product of the decrystallized cellulose (corresponding to 71% by weight on the basis of the cellulose-containing raw material charged). The resultant undersize product was subjected to measurements of an average particle size through the aforementioned method, and the crystallinity of the decrystallized cellulose was calculated from measured X-ray diffraction intensities. The results are shown in Table 2-1.

PRODUCTION EXAMPLE 2-3 (Reference Example)

The same procedure as described in Production Example 2-1 was repeated except that a vibration mill "Pot Mill ANZ-51S" available from Nitto Kagaku Co., Ltd., (container capacity: 1.0 L; 10 mmφ zirconia balls filled: 1.8 kg; filling ratio: 53%) was used in place of the batch-type agitation tank mill, and the pulp (100 g) as the cellulose-containing raw material was charged into the mill and treated therein at a rotating speed of 100 rpm for 48 hr, thereby obtaining decrystallized cellulose. After completion of the milling treatment, no pulp adhered on an inner wall surface or a bottom of the vibration mill was observed. The thus-obtained decrystallized cellulose was passed through a sieve having a mesh size of 75 μm, thereby obtaining 63 g of an undersize product of the decrystallized cellulose (corresponding to 63% by weight on the basis of the cellulose-containing raw material charged). The resultant undersize product was subjected to measurements of an average particle size through the aforementioned method, and the crystallinity of the decrystallized cellulose was calculated from measured X-ray diffraction intensities. The results are shown in Table 2-1.

[Table 11]

Table 2-1

|  |  | Production Ex. 2 - 1 | Production Ex. 2 - 2 | Production Ex. 2 - 3 |
|---|---|---|---|---|
| Shredding | | yes | yes | yes |
| Twin-screw extruder treatment | Treatment | yes | yes | yes |
| | Shear rate (sec⁻¹) | 660 | 660 | 660 |
| | Av. particle size (μm)*1 | 120 | 120 | 120 |
| | Bulk density (kg/m³) *1 | 219 | 219 | 219 |

(continued)

|  |  | Production Ex. 2 - 1 | Production Ex. 2 - 2 | Production Ex. 2 - 3 |
|---|---|---|---|---|
| Shredding |  | yes | yes | yes |
| Mill treatment | Treatment | yes | yes | yes |
|  | Kind of mill | Agitation tank mill | Vibration mill | Tumbling mill |
|  | Media | 5 mmφ zirconia | 20 mmφ zirconia | 10 mmφ zirconia |
|  | Amount of pulp charged (g) | 130 | 200 | 100 |
|  | Treatment time (hr) | 2.5 | 4 | 48 |
| Evaluation | Cellulose I-type Crystallinity (%) | 0 | 0 | 31 |
|  | Occurrence of deposit after milling[*2] | no | no | no |
|  | Ratio of weight passed through 75-μm sieve to total weight charged into mill (% by weight)[*3] | 90 | 71 | 63 |
|  | Av. particle size of decrystallized cellulose (μm) | 31 | 57 | 59 |
| [*1]: Average particle size or bulk density of pulp after twin-screw extruder treatment [*2]: Presence or absence of pulp adhered on the inner wall of the mill after the mill treatment [*3]: Weight of undersize product of decrystallized cellulose passing through a 75-μm sieve which was obtained after the mill treatment | | | | |

COMPARATIVE PRODUCTION EXAMPLE 2-1

The same procedure as described in Production Example 2-1 was repeated except for subjecting the pulp to the shredder treatment and then to the twin-screw extruder treatment, but no mill treatment was conducted, thereby obtaining a powdered pulp. The resultant powdered pulp was subjected to measurements of a bulk density and an average particle size, through the aforementioned method. The crystallinity of the obtained product was calculated from measured X-ray diffraction intensities. The results are shown in Table 2-2.

COMPARATIVE PRODUCTION EXAMPLE 2-2

The same shredder treatment as described in Production Example 2-1 was conducted to obtain a chipped pulp. Next, the resultant chipped pulp was charged into the batch-type agitation tank mill without previously subjecting the pulp to the extruder treatment. However, the amount of the chipped pulp capable of being charged into the mill was only 65 g which was one half of the pulp charged in Production Example 2-1, owing to a high bulkiness thereof. After completion of charging, the chipped pulp was subjected to the treatment using the batch-type agitation tank mill under the same conditions as employed in Production Example 2-1, thereby obtaining a powdered pulp. As a result, it was confirmed that after the treatment, the pulp adhered on the bottom of the agitation tank mill was observed. The thus obtained powdered pulp was passed through a sieve having a mesh size of 75 μm, thereby obtaining 16.9 g of the powdered pulp as an undersize product (corresponding to 26.0% by weight on the basis of the material charged). The resultant undersize product was subjected to measurements of an average particle size through the aforementioned method, and the crystallinity of the product was calculated from measured X-ray diffraction intensities. The results are shown in Table 2-2.

COMPARATIVE PRODUCTION EXAMPLE 2-3

The same shredder treatment as described in Production Example 2-1 was conducted to obtain a chipped pulp. Next, the resultant chipped pulp (500 g) was charged into a cutter mill "POWER MILL P-02S Model" available from Dalton Co., Ltd., without previously subjecting the pulp to the extruder treatment, and treated therein at a rotating speed of 3,000 rpm for 0.5 hr. The obtained pulp was in the form of a flocculated weight having a bulk density of 33 kg/m$^3$. Next, the flocculated pulp was charged into the batch-type agitation tank mill. However, the amount of the flocculated pulp capable of being charged into the mill was only 30 g owing to a high bulkiness thereof. After completion of charging, the flocculated pulp was subjected to the treatment using the batch-type agitation tank mill under the same conditions as employed in Production Example 2-1, thereby obtaining a powdered pulp. As a result, it was confirmed that after the treatment, no pulp adhered on the inside of the agitation tank mill was observed. The thus obtained powdered pulp was passed through a sieve having a mesh size of 75 μm, thereby obtaining 23.4 g of the powdered pulp as an undersize product (corresponding to 78.0% by weight on the basis of the material charged). The resultant undersize product was subjected to measurements of an average particle size and through the aforementioned method, and the crystallinity of the product was calculated from measured X-ray diffraction intensities. The results are shown in Table 2-2.

[Table 12]

Table 2-2 (1/2)

| | | | Comp. Production Exs. | | |
|---|---|---|---|---|---|
| | | | 2-1 | 2-2 | 2-3 |
| Shredding | | | yes | yes | yes |
| Twin-screw extruder treatment | Treatment | | yes | no | no |
| | Shear rate (sec$^{-1}$) | | 660 | - | - |
| | Av. particle size (μm) | | 120*1 | - | - |
| | Bulk (kg/m$^3$) | | 219*1 | - | 33*2 |
| Mill treatment | Treatment | | no | yes | yes |
| | Kind of mill | | - | Agitation tank mill | Agitation tank mill |
| | Media | | - | 5 mmφ zirconia | 5 mmφ zirconia |
| | Amount of pulp charged (g) | | - | 65 | 30 |
| | Treatment time (hr) | | - | 2.5 | 2.5 |
| Evaluation | Cellulose I-type Crystallinity (%) | | 76 | 0 | 0 |
| | Occurrence of deposit after milling*3 | | - | yes | no |
| | Ratio of weight passed through 75-μm sieve to total weight charged into mill (% by weight) *4 | | - | 26 | 78 |
| | Av. particle size of powdered pulp (μm) | | 156 | 38 | 23 |

*1: Average particle size or bulk density of pulp after twin-screw extruder treatment
*2: Bulk density of pulp after cutter mill treatment
*3: Presence or absence of pulp adhered on the inner wall of the mill after the mill treatment
*4: Weight of undersize product of decrystallized cellulose passing through a 75-μm sieve which was obtained after the mill treatment

Table 2-2 (2/2)

| | | Comp. Production Exs. | | | |
|---|---|---|---|---|---|
| | | 1-1 | 1-2 | 1-3 | 1-4 |
| Shredding | | yes | yes | yes | yes |
| Twin-screw extruder treatment | Treatment | no | no | no | no |
| | Shear rate (sec$^{-1}$) | - | - | - | - |
| | Av. particle size ($\mu$m) | - | - | - | - |
| | Bulk density (kg/m$^3$) | - | - | - | - |
| Mill treatment | treatment | yes | yes | yes | yes |
| | Kind of mill | Tumbling mill | Cutter mill | Hammer mill | Pin mill |
| | Media | 10 mm$\phi$ zirconia | - | - | - |
| | Amount of pulp charged (g) | 100 | 500 | 500 | 500 |
| | Treatment time (hr) | 48 | 0.5 | 0.5 | 0.25 |
| Evaluation | Cellulose I-type Crystallinity (%) | 73 | 78 | 74 | 75 |
| | Occurrence of deposit after milling*3 | - | - | - | - |
| | Ratio of weight passed through 75-$\mu$m sieve to total weight charged into mill (% by weight)*4 | - | - | - | - |
| | Av. particle size of powdered pulp ($\mu$m) | (Almost chip-like) | (Flocculated) | (Flocculated) | (Flocculated) |

*3: Presence or absence of pulp adhered on the inner wall of the mill after the mill treatment
*4: Weight of undersize product of decrystallized cellulose passing through a 75-$\mu$m sieve which was obtained after the mill treatment

Production Examples 2-4 to 2-8

Instead of pulp, cellulose-containing materials (wood-free paper (Production Example 2-4, cellulose content: 83% by weight and water content: 5.7% by weight), corrugated cardboard (Production Example 2-5, cellulose content: 84% by weight and water content 7.2% by weight), newspaper (Production Example 2-6, cellulose content: 83% by weight and water content: 7.7% by weight), rice straw (Production Example 2-7, cellulose content: 55% by weight and water content: 8.0% by weight), magazine (Production Example 2-8, cellulose content: 60% by weight and water content: 4.5% by weight) were subjected to the shredder treatment and the extruder treatment through the methods and under the conditions described Production Example 2-1. The properties of the cellulose-containing materials obtained after the extruder treatment are as follows: (Production Example 2-4) wood-free paper, average particle size of 71 $\mu$m/bulk density of 274 kg/m$^3$; (Production Example 2-5) corrugated cardboard, average particle size of 93 $\mu$m/bulk density of 216 kg/m$^3$; (Production Example 2-6) newspaper, average particle size of 61 $\mu$m/bulk density of 303 kg/m$^3$; (Production Example 2-7) rice straw, average particle size of 82 $\mu$m/bulk density of 339 kg/m$^3$, and (Production Example 2-8) magazine, average particle size of 72 $\mu$m/bulk density of 431 kg/m$^3$.
These materials were further subjected to the batch-type agitation-tank-mill treatment through the method and under the conditions described in Production Example 2-1, to thereby produce decrystallized celluloses. The properties thereof are as follows: (Production Example 2-4) wood-free paper, crystallinity of 0%/average particle size of 50 $\mu$m, (Production Example 2-5) corrugated cardboard, crystallinity of 0%/average particle size of 28 $\mu$m, (Production Example 2-6) newspaper, crystallinity of 0%/average particle size of 32 $\mu$m, (Production Example 2-7) rice straw, crystallinity of 2%/average particle size of 27 $\mu$m, and (Production Example 2-8) magazine, crystallinity of 4%/average particle size of 24 $\mu$m).

COMPARATIVE PRODUCTION EXAMPLE 2-4

Rice straw of Production Example 2-7 (cellulose content: 55% by weight; water content: 8.0% by weight) serving as the cellulose-containing raw material was subjected to the shredder treatment and extruder treatment through the methods and under the conditions described Production Example 2-1. However, no batch-type agitation-tank-mill treatment was performed, thereby obtaining a powdered cellulose (rice straw, crystallinity of 54%/average particle size of 82 $\mu$m).

EXAMPLES 2-1, Reference Example 2-2 and COMPARATIVE EXAMPLE 2-1

In Example 2-1, the decrystallized cellulose (crystallinity of 0%/average particle size of 43 $\mu$m) produced in Production Example 2-1 was employed as a substrate. In Reference Example 2-2, there was employed a decrystallized cellulose (crystallinity of 30%/average particle size of 46 $\mu$m) produced in the same manner as employed in Production Example 2-1, except for the mill treatment time was changed from 2.5 hours to 30 minutes. In Comparative Example 2-1, a commercial powdered cellulose product (KC Flock, product of Nippon Paper Chemicals Co., Ltd., crystallinity of 76%, particle size of 26 $\mu$m) was employed. Each of the samples was subjected to saccharification reaction by use of a cellulases standard sample (Celluclast 1.5L, product of Novozymes). Each of the decrystallized celluloses and powdered pulp products (0.15 g) was suspended in an enzymatic reaction mixture (3 mL), and the mixture was caused to react in at 50°C for 24 hours, 48 hours, and 72 hours, during which the mixture was stirred with shaking. The enzymatic reaction mixture employed was 100mM citrate buffer (pH: 5.0), 1% (v/v) Celluclast 1.5L (0.17% as protein), and 30 $\mu$g/mL tetracycline). After completion of reaction, the reaction mixture was separated into a precipitate and a supernatant through centrifugation. The weight of dried precipitate was measured. The amount of reducing saccharide (as glucose) released to the supernatant was quantitatively determined through the phenol-sulfuric acid method. The results are shown in Table 2-3.

[Table 13]

Table 2-3

| | Sample | Crystallinity (%) | Amount of saccharide released in supernatant (glucose base) (g/L) (Weight of dried precipitate(g/L) | | | |
|---|---|---|---|---|---|---|
| | | | Reaction time (hr) | | | |
| | | | 0 (unreacted) | 24 | 48 | 72 |
| Ex. 2-1 | Decrystallized cellulose | 0 | 1.5 (52.1) | 18.7 (26.6) | 47.1 (5.6) | 46.0 (2.7) |
| Reference Ex. 2-2 | Decrystallized cellulose | 30 | 0.9 (55.8 | 16.2 (31.0) | 31.9 (26.3) | 51.7 (14.7) |
| Comp. Ex. 2-1 | Powdered cellulose commercial product | 76 | 0.2 (60.2) | 14.7 (45.8) | 25.4 (44.1) | 26.3 (36.9) |

EXAMPLES 2-3 to 2-5 and COMPARATIVE EXAMPLES 2-2 to 2-4

In Examples 2-3 to 2-5, decrystallized cellulose was prepared under the aforementioned conditions of Production Example 2-1 (crystallinity of 0%/average particle size of 43 $\mu$m). In Comparative Examples 2-2 to 2-4, a powdered pulp product (crystallinity of 76%/average particle size of 156 $\mu$m) prepared in Comparative Production Example 2-1 was employed. Each of the samples was subjected to saccharification reaction by use of a cellulases standard sample (Celluclast 1.5L, product of Novozymes). The decrystallized cellulose or powdered pulp product (0.15 g) was suspended in an enzymatic reaction mixture (3 mL), and the mixture was caused to react at 50°C for 6 hours, 24 hours, 48 hours, and 72 hours, during which the mixture was stirred with shaking. The following enzymatic reaction mixtures were employed: (Example 2-3 and Comparative Example 2-2) 100mM citrate buffer (pH: 5.0), 3% (v/v) Celluclast 1.5L (0.5% as protein), and 30 $\mu$g/mL tetracycline); (Example 2-4 and Comparative Example 2-3) 100mM citrate buffer (pH: 5.0), 1.5% (v/v) Celluclast 1.5L (0.25% as protein), and 30 $\mu$g/mL tetracycline); and (Example 2-5 and Comparative Example 2-4) 100mM citrate buffer (pH: 5.0), 0.6% (v/v) Celluclast 1.5L (0.1% as protein), and 30 $\mu$g/mL tetracycline). After completion of reaction, the reaction mixture was separated into a precipitate and a supernatant through centrifugation. The amount of reducing saccharide (as glucose) released to the supernatant was quantitatively determined through the DNS method. The results are shown in Table 2-4.

[Table 14]

Table 2-4

| | Sample | Crystallinity (%) | Amount of reducing saccharide released In supernatant (g/L) | | | |
|---|---|---|---|---|---|---|
| | | | Reaction time (hr) | | | |
| | | | 6 | 24 | 48 | 72 |
| Ex. 2-3 | Decrystallized cellulose | 0 | 19.0 | 37.6 | 46.1 | 50.7 |
| Comp. Ex. 2-2 | Powdered pulp | 76 | 13.4 | 22.5 | 31.1 | 34.3 |
| Ex. 2-4 | Decrystallized cellulose | 0 | 13.6 | 33.9 | 38.3 | 46.1 |
| Comp. Ex. 2-3 | Powdered pulp | 76 | 8.8 | 18.4 | 23.1 | 28.1 |
| Ex. 2-5 | Decrystallized cellulose | 0 | 13.0 | 28.5 | 35.1 | 41.7 |
| Comp. Ex. 2-4 | Powdered pulp | 76 | 6.1 | 15.0 | 21.0 | 20.8 |

EXAMPLE 2-6

The supernatant of the saccharification mixture (reaction time: 72 hours) obtained in Example 2-3 was analyzed in the following manner by means of a DX500 Chromatography System (Dionex Corporation) (column: CarboPac PA1 (Dionex Corporation, 4 × 250 mm) and detector: ED40 pulsed amperometry detector). The following eluents were employed: (A) 100mM sodium hydroxide solution, (B) 100mM sodium hydroxide solution containing 1M sodium acetate, and (C) ultrapure water.

Sugar analysis was performed under linear gradient conditions: A 10%-C 90% (at injection) and A 95%-B 5% (0 to 15 min). As standards, 0.01% (w/v) glucose (Wako Pure Chemical Industries, Ltd.), xylose (Wako Pure Chemical Industries, Ltd.), xylobiose (Wako Pure Chemical Industries, Ltd.), and cellobiose (Seikagaku Corporation). The retention times of the standards are as follows: glucose (about 5.5 min), xylose (about 6.5 min), xylobiose (about 14 min), and cellobiose (about 14.5 min). Each supernatant of the saccharification mixture was 100-fold diluted, and an aliquot (10 $\mu$L) was injected. The results are shown in Table 2-5.

[Table 15]

Table 2-5

| | Sample | Amount of saccharide released in supernatant (g/L) | | | |
|---|---|---|---|---|---|
| | | Reaction time (72 hr) | | | |
| | | glucose | xylose | xylobiose | cellobiose |
| Ex. 2-6 | Decrystallized cellulose | 41 | 3.7 | 3.2 | 4.8 |

EXAMPLES 2-7 to 2-9 and COMPARATIVE EXAMPLES 2-5 to 2-7

In Examples 2-7 to 2-9, decrystallized celluloses produced in Production Examples 2-4 to 2-6 ((Example 2-7) wood-free paper, crystallinity of 0%/average particle size of 50 $\mu$m, (Example 2-8) corrugated cardboard, crystallinity of 0%/average particle size of 28 $\mu$m, and (Example 2-9) newspaper, crystallinity of 0%/average particle size of 32 $\mu$m) were employed. In Comparative Examples 2-5 to 2-7, powdered celluloses produced in Comparative Production Examples 1-6 to 1-8 ((Comparative Example 2-5) wood-free paper, crystallinity of 71%/average particle size of 71 $\mu$m, (Comparative Example 2-6) corrugated cardboard, crystallinity of 71%/average particle size of 93 $\mu$m, and (Comparative Example 2-7 newspaper, crystallinity of 56%/average particle size of 61 $\mu$m) were employed. In the Examples and Comparative Examples, each of the cellulose products was subjected to saccharification reaction by use of a cellulases standard product (Celluclast 1.5L, Novozymes). Each of the decrystallized celluloses and powdered celluloses (0.15 g) was suspended in an enzymatic reaction mixture (3 mL), and the mixture was caused to react at 50°C for 6 hours, 24 hours, 48 hours, and 72 hours, during which the mixture was stirred with shaking. The following enzymatic reaction mixtures were employed: (Examples 2-7 and 2-8 and Comparative Examples 2-5 and 2-6) 100mM citrate buffer (pH: 5.0), 1.5% (v/v) Celluclast 1.5L (0.25% as protein), and 30 μg/mL tetracycline and (Example 2-9 and Comparative Example 2-7) 100mM citrate buffer (pH: 5.0), 12% (v/v) Celluclast 1.5L (2% as protein), and 30 μg/mL tetracycline. After completion of reaction, the reaction mixture was separated into a precipitate and a supernatant through centrifugation. The amount of reducing saccharide (as glucose) released to the supernatant was quantitatively determined through the DNS method. The results are shown in Table 2-6.

[Table 16]

Table 2-6

| | Cellulose-containing raw material | Sample | Crystallinity (%) | Amount of reducing saccharide released in supernatant (g/L) | | | |
|---|---|---|---|---|---|---|---|
| | | | | Reaction time (hr) | | | |
| | | | | 6 | 24 | 48 | 72 |
| Ex. 2-7 | Wood-free paper | Decrystallized cellulose | 0 | 9.7 | 15.1 | 22.8 | 25.5 |
| Comp. Ex. 2-5 | | Powdered cellulose | 71 | 10.6 | 13.3 | 18.7 | 21.8 |
| Ex. 2-8 | Corrugated cardboard | Decrystallized cellulose | 0 | 17.7 | 26.8 | 26.7 | 28.6 |
| Comp. Ex. 2-6 | | Powdered cellulose | 71 | 8.8 | 12.3 | 13.2 | 13.1 |
| Ex. 2-9 | Newspaper | Decrystallized cellulose | 0 | 20.9 | 27.1 | 30.2 | 34.6 |
| Comp. Ex. 2-7 | | Powdered cellulose | 56 | 12.7 | 9.3 | 11.9 | 13.2 |

EXAMPLE 2-10 and COMPARATIVE EXAMPLE 2-8

In Example 2-10, decrystallized cellulose (rice straw, crystallinity of 2%/average particle size of 27 μm) obtained in Production Example 2-7 was employed. In Comparative Example 2-8, a powdered cellulose (rice straw, crystallinity of 54%/average particle size of 82 μm) obtained in Comparative Production Example 2-4 was employed. In the Example and Comparative Example, each of the cellulose products was subjected to saccharification reaction by use of a cellulases standard product (Celluclast 1.5L, Novozymes). The decrystallized cellulose or powdered cellulose (0.15 g) was suspended in an enzymatic reaction mixture (3 mL), and the mixture was caused to react at 50°C for 3 days, during which the mixture was stirred with shaking. The enzymatic reaction mixture employed was 100mM citrate buffer (pH: 5.0), 3% (v/v) Celluclast 1.5L (0.5% as protein), and 30 μg/mL tetracycline. After completion of reaction, the reaction mixture was separated into a precipitate and a supernatant through centrifugation. The amount of reducing saccharide (as glucose) released to the supernatant was quantitatively determined through the DNS method. The results are shown in Table 2-7.

[Table 17]

Table 2-7

| | Cellulose-containing raw material | Sample | Crystallinity (%) | Amount of reducing saccharide released in supernatant (g/L) |
|---|---|---|---|---|
| | | | | Reaction time (72 hr) |
| Ex. 2-10 | rice straw | decrystallized cellulose | 2 | 31.3 |
| Comp. Ex. 2-8 | | powdered cellulose | 54 | 13.1 |

EXAMPLES 2-11 and 2-12

In Examples 2-11 and 2-12, decrystallized celluloses produced in Production Examples 2-6 and 2-8 ((Example 2-11) newspaper, crystallinity of 0%/average particle size of 32 $\mu$m and (Production Example 2-12) magazine, crystallinity of 4%/average particle size of 24 $\mu$m) were employed. In the Examples, each cellulose product was subjected to saccharification reaction by use of a cellulases standard product (Celluclast 1.5L, Novozymes). The decrystallized cellulose or powdered cellulose (0.15 g) was suspended in an enzymatic reaction mixture (3 mL), and the mixture was caused to react at 50°C for 3 days, during which the mixture was stirred with shaking. The following enzymatic reaction mixtures were employed: (Example 2-11) 100mM acetate buffer (pH: 3.5), 3% (v/v) Celluclast 1.5L (0.5% as protein), and 30 $\mu$g/mL tetracycline and (Example 2-12) 200mM acetate buffer (pH: 3.5), 3% (v/v) Celluclast 1.5L (0.5% as protein), and 30 $\mu$g/mL tetracycline. After completion of reaction, the reaction mixture was separated into a precipitate and a supernatant through centrifugation. The amount of reducing saccharide (as glucose) released to the supernatant was quantitatively determined through the DNS method. The results are shown in Table 2-8.

[Table 18]

Table 2-8

| | Cellulose-containing raw material | Sample | Crystallinity (%) | Amount of reducing saccharide released in supernatant (g/L) |
|---|---|---|---|---|
| | | | | Reaction time (72 hr) |
| Ex. 2-11 | newspaper | decrystallized cellulose | 0 | 34.9 |
| Ex. 2-12 | magazine | decrystallized cellulose | 4 | 27.9 |

From Tables 2-1 and 2-2, it was confirmed that the processes for production of the decrystallized celluloses according to Production Examples 2-1 to 2-3 were capable of producing decrystallized cellulose having reduced crystallinity in an efficient manner and were therefore excellent in productivity as compared to those according to Comparative Production Examples 2-1 to 2-3 and 1-1 to 1-4. In addition, the decrystallized celluloses produced in Production Examples 2-1 to 2-3 were found to have an appropriate average particle size, as compared with those produced in Comparative Production Example 2-1.

As is clear from Tables 2-3 and 2-4, the process for producing saccharide shown in Examples 2-1 to 2-5 exhibited higher productivity as compared with Comparative Examples 2-1 to 2-4. As is clear from Table 2-5, the produced saccharide was mainly formed of glucose. Thus, the process of the present invention is useful for the production of a raw material for producing ethanol or lactic acid through fermentation. As is clear from Tables 2-6, 2-7 and 2-8, the process for producing Saccharide is effective for various cellulose materials; i.e., not only for pulp but also for paper materials such as wood-free paper, corrugated cardboard, newspaper and magazine; and plant stems and leaves such as rice straw.

INDUSTRIAL APPLICABILITY

According to the process of the present invention for producing saccharide, decrystallized cellulose having reduced cellulose I-type crystallinity can be produced from a cellulose-containing raw material through a preliminary treatment. Therefore, saccharide can be produced at high productivity and efficiency through enzymatic reaction. The thus-produced saccharide is useful for, for example, production of substances such as ethanol and lactic acid through fermentation or a similar method.

**Claims**

1. A process for producing saccharide, including saccharifying decrystallized cellulose prepared from a raw material containing cellulose having cellulose I-type crystallinity of more than 33% as calculated from the following formula (1):

$$\text{Cellulose I-type Crystallinity (\%)} = [(I_{22.6} - I_{18.5}) / I_{22.6}] \times 100 \qquad (1),$$

wherein $I_{22.6}$ is diffraction intensity of a lattice plane (002 plane) as measured at a diffraction angle $2\theta$ of 22.6° in X-ray diffraction analysis; and $I_{18.5}$ is diffraction intensity of an amorphous moiety as measured at a diffraction angle 2θ of 18.5° in X-ray diffraction analysis, the process comprising:

treating the cellulose-containing raw material by means of a mill to reduce the cellulose I-type crystallinity of the cellulose to 10% or less, wherein the cellulose-containing raw material has a cellulose content of a residue obtained by removing water from the cellulose-containing raw material of 20% by weight or more, to thereby prepare decrystallized cellulose, and
causing a cellulase and/or a hemicellulase to act on the decrystallized cellulose, to thereby saccharify the decrystallized cellulose,
wherein the cellulose-containing raw material has a bulk density of 100 to 500 kg/m$^3$ and an average particle size of 0.01 to 1 mm.

2. A process for producing saccharide according to claim 1, wherein the cellulose-containing raw material is treated by means of a mill for 0.01 to 72 hours.

3. A process for producing saccharide according to claim 1 or 2, wherein the cellulose-containing raw material is a material which has been preliminarily treated by means of an extruder.

4. A process for producing saccharide according to claim 3, wherein the extruder is a twin-screw extruder.

5. A process for producing saccharide according to any of claims 1 to 4, wherein the mill is a media-type mill.

6. A process for producing saccharide according to any of claims 1 to 5, wherein the cellulose-containing raw material is at least one species selected from the group consisting of pulp, paper, stems or leaves of plants, plant shells, and wood.

**Patentansprüche**

1. Verfahren zur Erzeugung eines Saccharides, umfassend das Verzuckern von dekristallisierter Cellulose, hergestellt aus einem Ausgangsmaterial, umfassend Cellulose mit einer Cellulose-I-Typ-Kristallinität von mehr als 33%, berechnet anhand der folgenden Gleichung (1):

$$\text{Cellulose-I-Typ-Kristallinität (\%)} = [(I_{22,6} - I_{18,5})/I_{22,6}] \times 100 \qquad (1)$$

worin $I_{22,6}$ die Beugungsintensität einer Gitterebene (002-Ebene) ist, gemessen bei einem Beugungswinkel 2θ von 22,6° bei der Röntgenbeugungsanalyse; und $I_{18,5}$ die Beugungsintensität eines amorphen Anteils ist, gemessen bei einem Beugungswinkel 2θ von 18,5° bei der Röntgenbeugungsanalyse, wobei das Verfahren umfasst:
Behandlung des Cellulose-haltigen Ausgangsmaterials mit Hilfe einer Mühle, zur Verminderung der Kristallinität von Cellulose-I-Typ der Cellulose auf 10% oder weniger, worin das Cellulose-haltige Ausgangsmaterial einen Cellulosegehalt eines Restes hat, erhalten durch Entfernen von Wasser von dem Cellulose-haltigen Ausgangsmaterial von 20 Gew.-% oder mehr, zur Herstellung einer dekristallisierten Cellulose, und
Verursachen, dass eine Cellulase und/oder eine Hemicellulase auf die dekristallisierte Cellulose wirkt, um hierdurch die dekristallisierte Cellulose zu verzuckern,
worin das Cellulose-haltige Ausgangsmaterial eine Schüttdichte von 100-500 kg/m$^3$ und eine durchschnittliche Teilchengröße von 0,01-1 mm hat.

2. Verfahren zur Erzeugung eines Saccharides nach Anspruch 1, worin das Cellulose-haltige Ausgangsmaterial mit Hilfe einer Mühle für 0,01 bis 72 Stunden behandelt wird.

3. Verfahren zur Erzeugung von Saccharid nach Anspruch 1 oder 2, worin das Cellulose-haltige Ausgangsmaterial ein Material ist, das zuvor mit Hilfe eines Extruders behandelt ist.

4. Verfahren zur Erzeugung eines Saccharides nach Anspruch 3, worin der Extruder ein Doppelschraubenextruder ist.

5. Verfahren zur Erzeugung eines Saccharides nach einem der Ansprüche 1 bis 4, worin die Mühle eine Mühle vom Medientyp ist.

**6.** Verfahren zur Erzeugung von Saccharid nach einem der Ansprüche 1 bis 5, worin das Cellulose-haltige Ausgangsmaterial zumindest eine Spezies ist, ausgewählt aus der Gruppe bestehend aus Pulpe, Papier, Stämmen oder Blättern von Pflanzen, Pflanzenhüllen und Holz.

**Revendications**

**1.** Processus de production de saccharide comportant la saccharification de cellulose dé-cristallisée préparée à partir d'une matière première contenant de la cellulose avec une cristallinité de type cellulose I de plus de 33% comme calculé selon la formule (1) suivante :

$$\texttt{Cristallinité de type Cellulose I (\%) = [(I_{22,6}-I_{18,5})/I_{22,6}]\times 100 (1),}$$

où $I_{22,6}$ est l'intensité de diffraction d'un plan réticulaire (plan 002) telle que mesurée à un angle de diffraction $2\theta$ de 22,6° dans une analyse de diffraction de rayons X ; et $I_{18,5}$ est l'intensité de diffraction d'un fragment amorphe telle que mesurée à un angle de diffraction $2\theta$ de 18,5° dans une analyse de diffraction de rayons X, le processus comprenant le fait :

de traiter la matière première contenant de la cellulose au moyen d'un broyeur afin de réduire la cristallinité de type cellulose I de la cellulose à 10% ou moins, où la matière première contenant de la cellulose a une teneur en cellulose d'un résidu obtenu en éliminant l'eau de la matière première contenant de la cellulose de 20% en poids ou plus, pour préparer ainsi de la cellulose dé-cristallisée, et
d'amener une cellulase et/ou une hémicellulase à agir sur la cellulose dé-cristallisée, pour ainsi saccharifier la cellulose dé-cristallisée,
où la matière première contenant de la cellulose a une densité apparente de 100 à 500 kg/m$^3$ et une dimension moyenne des particules de 0,01 à 1 mm.

**2.** Processus de production de saccharide selon la revendication 1, dans lequel la matière première contenant de la cellulose est traitée au moyen d'un broyeur pour 0,01 à 72 heures.

**3.** Processus de production de saccharide selon la revendication 1 ou 2, dans lequel la matière première contenant de la cellulose est une matière qui a été préalablement traitée au moyen d'une extrudeuse.

**4.** Processus de production de saccharide selon la revendication 3, dans lequel l'extrudeuse est une extrudeuse à double vis.

**5.** Processus de production de saccharide selon l'une des revendications 1 à 4, dans lequel le broyeur est un broyeur du type à milieu.

**6.** Processus de production de saccharide selon l'une des revendications 1 à 5, dans lequel la matière première contenant de la cellulose est au moins une espèce choisie dans le groupe constitué de pulpe, de papier, de tiges ou de feuilles de plantes, d'écorces de plantes, et de bois.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006223152 A
- JP 62236801 A
- JP 2003064184 A
- JP 2004331918 A
- JP 2005068140 A
- JP 2003135052 A
- JP 2007074992 A
- JP 2007074993 A

**Non-patent literature cited in the description**

- **ZHANG et al.** *Cellulose,* 2007, vol. 14, 447-456
- **KOBAYASHI et al.** *Powder Technology,* 2007, vol. 180, 272-283
- **KOBAYASHI et al.** *Journal of the Japan Institute of Energy,* 2007, vol. 86, 730-735
- **CHRISTAKOPOULOS et al.** *Enzyme Microb. Technol.,* 1991, vol. 13, 272-4
- **TASSINARI et al.** *Biotechnology and Bioengineering,* 1980, vol. XXII, 1689-1705
- Progress of Chemical Engineering; 30th Collection; Control of Microparticles. Institute of Chemical Engineering, 10 October 1996